# FASCICULE DE BREVET EUROPEEN

(11) **EP 2 083 923 B1**
(45) Date de publication et mention de la délivrance du brevet: **05.05.2010**
(21) Numéro de dépôt: 07858560.1
(22) Date de dépôt: 12.10.2007
(51) Int. Cl.: A61Q 5/06

(54) **COMPOSITION COMPRENANT UN COLORANT DE TYPE STYRYLIQUE OU IMINIQUE ET UN COMPOSE THIOLE, PROCEDE DE COLORATION ET DISPOSITIF**
ZUSAMMENSETZUNG AUS EINEM FARBSTOFF VOM STYRYL- ODER IMIN-TYP UND EINER THIOL-VERBINDUNG, FÄRBEVERFAHREN UND VORRICHTUNG
COMPOSITION COMPRISING A DYE OF STYRYL OR IMINE TYPE AND A THIOL COMPOUND, DYEING PROCESS AND DEVICE

(30) Priorité: 13.10.2006 FR 0654260
(43) Date de publication de la demande: 05.08.2009
(73) Titulaire: L'Oréal, 75008 Paris (FR)
(72) Inventeur: PLOS, Grégory, 75018 Paris (FR)
(74) Mandataire: Wattremez, Catherine
(86) Numéro de dépôt international: PCT/FR2007/052130
(87) Numéro de publication internationale: WO 2008/043968

(56) Documents cités:
- EP-A- 1 655 056
- EP-A- 1 747 774
- JP-A- 55 113 710
- JP-A- 56 150 006
- US-A- 4 314 058
- US-A- 4 380 629

## Description

La présente invention a pour objet une composition pour la coloration des fibres kératiniques, et en particulier des fibres kératiniques humaines telles que les cheveux, comprenant au moins un colorant de type styrylique ou iminique et au moins un composé thiolé. Elle concerne de plus un procédé de coloration mettant en oeuvre une telle composition, ainsi qu'un dispositif approprié pour la mise en oeuvre dudit procédé.

Il est connu de teindre les fibres kératiniques, et en particulier les fibres kératiniques humaines telles que les cheveux, avec des compositions tinctoriales contenant des précurseurs de colorants d'oxydation, appelés généralement bases d'oxydation, tels que des ortho- ou para-phénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, donnent naissance par un processus de condensation oxydative, à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

Un autre avantage de ce type de coloration est d'être visible sur cheveux foncés. En effet, le processus oxydatif étant réalisé en milieu alcalin, le plus généralement en présence d'ammoniaque, il se produit, parallèlement à la condensation des précurseurs de coloration, une décoloration de la mélanine, pigment naturel des fibres kératiniques et notamment les cheveux. La couleur obtenue peut donc aussi être visible même sur cheveux foncés.

Les colorations obtenues présentent une bonne ténacité notamment vis-à-vis des shampoings. Cependant, il est rare de pouvoir obtenir par cette méthode des colorations chromatiques.

Il est aussi connu de teindre les fibres kératiniques avec des compositions contenant des colorants directs. Ces composés sont des molécules colorées et colorantes qui ont une affinité pour les fibres kératiniques. Ils sont appliqués sur les fibres pendant un temps nécessaire à l'obtention de la coloration désirée, puis généralement rincés.

Les colorants directs classiquement utilisés sont en particulier des colorants du type nitré benzénique, anthraquinonique, nitropyridinique, azoïque, azoïque cationique, xanthénique, acridinique, azinique, triarylméthane ou des colorants naturels.

Il est possible d'obtenir des colorations plus claires que celles du cheveu avant la coloration, si l'on effectue la coloration directe en présence d'un agent oxydant. On parle alors de coloration directe en conditions éclaircissantes.

Les colorations directes permettent d'atteindre avantageusement des couleurs très chromatiques mais elles présentent toujours l'inconvénient d'être temporaires ou semi-permanentes. En effet, la ténacité des colorants directs sur le cheveu reste limitée, ce qui conduit à un affadissement de la couleur, voire à un virage de celle-ci au cours du temps, dus au départ de l'un ou plusieurs des colorants employés.

Un inconvénient de ces deux modes de coloration est la nécessité d'employer une composition oxydante pour la coloration d'oxydation ou pour obtenir une coloration directe éclaircie. Or il est connu que les compositions oxydantes, à la longue, provoquent une dégradation de la fibre capillaire.

Un autre inconvénient de ces deux modes de coloration réside dans le fait que les compositions sont initialement colorées, comme dans le cas de compositions de coloration mettant en jeu des colorants directs, ou bien le deviennent durant l'application, comme c'est le cas des compositions comprenant un ou plusieurs précurseurs de colorants d'oxydation. Par conséquent, la coloration directe et la coloration d'oxydation présentent le désavantage d'être salissantes.

On a récemment décrit, dans la demande française FR 05 52277, la mise en oeuvre de composés de type styrylique ou iminique existants sous une forme colorée et sous une forme incolore, pour la coloration de fibres kératiniques. Ainsi, dans certaines conditions, la composition mise en oeuvre est incolore ou faiblement colorée et la couleur est révélée dans les fibres kératiniques une fois l'application faite, ce qui permet de résoudre le problème de tachage de la peau et des tissus employés.

Si ces compositions apportent de nombreux avantages par rapport aux compositions existantes, tout en offrant une efficacité satisfaisante de coloration, il n'en reste pas moins que l'on peut souhaiter améliorer encore la montée et diminuer la sélectivité de la coloration obtenue.

La présente invention permet de plus d'accéder à des colorations des fibres kératiniques plus claires que la couleur initiale sans avoir recours nécessairement à une composition oxydante.

Enfin, le procédé selon l'invention permet dans certaines conditions, de proposer un procédé qui ne tache pas.

Ces buts et d'autres sont atteints par la présente invention qui a pour objet une composition pour la coloration des fibres kératiniques, comprenant, dans un milieu approprié pour la teinture, a) au moins un composé de formule (1) et ses sels d'addition : 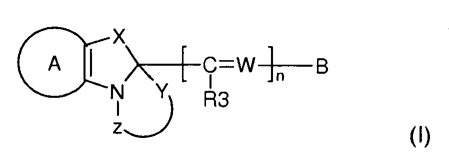 dans laquelle :
- A est un noyau aromatique ou hétéroaromatique, condensé ou non, comportant de 5 à 16 chaînons, non substitué ou substitué ;
- X représente un atome d'oxygène, un atome de soufre ou un groupement CR₁R₂ ;
- R₁ et R₂ représentent, indépendamment l'un de l'autre, un atome d'hydrogène, un radical alkyle en C₁-C₆, un radical hydroxyalkyle en C₁-C₆, un radical alcoxyalkyle en C₁-C₆, une chaîne alkylène pouvant contenir un atome d'oxygène ou de soufre ; R₁ et R₂ peuvent former ensemble un cycle à 5 ou 6 chaînons aromatiques ou non aromatiques, contenant éventuellement un ou plusieurs hétéroatomes tels qu'un atome d'azote, d'oxygène ou de soufre ;
- R₃ représente un atome d'hydrogène, un atome d'halogène, un radical alkyle en C₁-C₆, un radical cyano, un groupement aromatique, un groupement phénoxy, un radical nitro ;
- W représente un groupement CR₄ ou un atome d'azote ;
- R₄ représente un atome d'hydrogène, un atome d'halogène, un radical alkyle en C₁-C₆, un radical cyano, un groupement aromatique, un radical phénoxy, un radical nitro ;
- Y représente un atome d'oxygène, un atome de soufre, un groupement NR₅ ;
- R₅ représente un atome d'hydrogène, un radical alkyle en C₁-C₆ ;
- Z représente un groupement -CₚH₂ₚ-, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ; un groupement -C_{q}H_{2q}CO-, avec q un entier compris entre 1 et 3, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ;
- n représente un entier de 1 à 4 ;
- B représente un noyau aromatique ou hétéroaromatique, condensé ou non, comportant de 5 à 16 chaînons non substitué ou substitué,
Et b), au moins un composé thiolé comprenant au moins une fonction thiol (SH) et un groupement comprenant de 1 à 20 atomes de carbone, saturé ou non, ledit groupement étant éventuellement interrompu par un ou plusieurs groupes non adjacents (séparés par au moins un atome de carbone) choisis parmi -O-, -S-, -S-S-, amino (-NR-), carbonyle (-CO-), oxycarbonyle (-O-CO-), aminocarbonyle (-NR-CO-), noyau aromatique ou hétéroaromatique, les groupements amino étant ou non substitués par un ou deux radicaux alkyle en C₁-C₆ à la condition que l'atome de soufre de la fonction thiol soit rattaché audit groupement au moyen d'un atome de carbone ; ledit groupement étant éventuellement substitué par un ou plusieurs hydroxyle, alcoxy en C₁-C₆, hydroxycarbonyle, alcoxy(C₁-C₆)carbonyle, amino, aminocarbonyle, alkylcarbonylamino, dans lesquels la fonction amino est substituée ou non par un ou deux radicaux alkyle en C₁-C₆.

La présente invention a également pour objet un procédé de coloration des fibres kératiniques mettant en oeuvre une telle composition.

La présente invention a aussi pour objet un dispositif à plusieurs compartiments pour la mise en oeuvre du procédé de coloration conforme à l'invention.

L'invention permet en particulier d'obtenir une coloration des fibres kératiniques qui est chromatique, tenace, notamment vis-à-vis des shampooings.

Comme indiqué auparavant, un autre avantage de l'invention est de fournir un mode de coloration propre, en d'autres termes qui ne tache pas.

En effet, dans certaines conditions de mise en oeuvre de l'invention, le composé appliqué sur les fibres kératiniques est incolore ou faiblement coloré et la coloration est n'est révélée que par la suite, une fois l'application sur les fibres kératiniques, réalisée.

La révélation de la coloration est effectuée en ouvrant l'hétérocycle pour conduire à des espèces de formule (1') suivante, qui elles sont colorées :

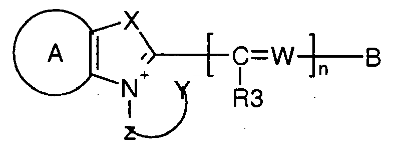

A, X, Z, W, R3 et n ayant les mêmes significations que celles indiquées auparavant.

L'ouverture de l'hétérocycle formé par N, Y et Z dans les composés colorants de formule (1) peut être réalisée sous l'effet d'un stimulus tel que la lumière, un courant électrique, la chaleur, l'ajout d'un agent acidifiant, l'ajout de solvant ou une radiation électromagnétique.

La composition appliquée sur les fibres kératiniques est donc substantiellement (ou sensiblement) incolore, transparente ou non, de même que les eaux de rinçage à l'eau ou au shampooing, rendant de ce fait. Ainsi, l'application de la composition selon l'invention peut ne pas être salissante.

Par ailleurs, il est possible d'effacer la couleur obtenue. Il suffit en effet de traiter les fibres kératiniques colorées par la composition selon l'invention, avec une composition qui aura pour rôle de faire augmenter le pH des fibres au-dessus du pKa des colorants de formules (I) présents sur et dans les fibres. Les hétérocycles ouverts des composés de formule (I') se refermeront pour donner à nouveau des composés de formule (I).

On a enfin constaté que l'application de la composition sur des cheveux foncés, plus particulièrement caractérisés par une hauteur de ton inférieure ou égale à 6, conduisait à augmenter la hauteur de ton de la coloration d'au moins un ton, ce qui correspond à un éclaircissement visible de la fibre, sans avoir recours à l'emploi d'une composition oxydante.

Dans le cadre de la présente invention, on entend par noyau hétéroaromatique un noyau aromatique comprenant un ou plusieurs hétéroatomes tels que les atomes d'azote, de soufre, d'oxygène ou de phosphore.

Dans le cadre de la présente invention, le terme "condensé" signifie au moins deux cycles accolés présentant au moins deux atomes communs.

Un radical halogéno désigne un atome d'halogène choisi parmi le chlore, le brome, l'iode et le fluor.

On entend par radical alkyle (alk) un radical linéaire ou ramifié, par exemple un radical méthyle, éthyle, n-propyle, iso-propyle, n-butyle ou ter-butyle. Un radical alcoxy est un radical alk-O-, un radical mono ou dialkylamino est un radical -N(alk)ₙ avec n = 1 ou 2, un radical alkylcarbonyle est un radical alk-CO-, un radical alcoxycarbonyle est un radical alk-O-CO-, un radical alkylcarbonylalkyle est un radical alk-CO-alk-, dans chacune de ces définitions le radical alkyle étant tel que défini précédemment.

Un radical alkyle substitué est un alkyle mono ou polysubstitué. Par exemple, un hydroxyalkyle ou un aminoalkyle est un alkyle qui peut être substitué par un ou plusieurs groupes hydroxy ou amino.

On entend par radical aryle (ar) un radical carboné dérivé des composés benzéniques condensés ou non condensés, par exemple phényle, anthracényle ou naphtyle.

A titre d'exemples de cycles à 5 ou 6 chaînons aromatiques ou non aromatiques, on peut citer le cyclo-1,3-pentadiène, le benzène, le cyclopentane et le cyclobutane.

Les composés de formule (I) peuvent être neutralisés par un contre-ion anionique ou cationique quand ils portent une charge. Les contre-ions négatifs peuvent par exemple être choisis parmi un halogénure tel qu'un chlorure, un bromure, un iodure ou un fluorure, un perchlorate, un p-méthylbenzène sulfonate, un tétrafluoroborate, un sulfate, un alkylsulfate, un toluènesulfonate, un sulfonate. Les contre-ions cationiques peuvent être choisis parmi les cations issus des sels de métaux alcalins ou alcalino-terreux tels que les ions sodium ou potassium.

Dans le cadre de la présente invention, on entend par cheveux foncés, des cheveux dont la hauteur de ton est inférieure ou égale à 6 (blond foncé) et de préférence inférieure ou égale à 4 (châtain).

Il est rappelé que l'éclaircissement des cheveux est évalué par la hauteur de ton" qui caractérise le degré ou le niveau d'éclaircissement. La notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage "Sciences des traitements capillaires" de Charles ZVIAK 1988, Ed.Masson, pp.215 et 278. Les hauteurs de ton s'échelonnent de 1 (noir) à 10 (blond clair), une unité correspondant à un ton ; plus le chiffre est élevé et plus la nuance est claire.

Selon un mode de réalisation particulier de l'invention, A est un noyau benzénique, anthracénique, naphtalénique ou quinolinique.

Selon un mode de réalisation particulier de l'invention, A est non substitué ou substitué par un ou plusieurs groupements pouvant être choisis parmi un radical halogéno, un radical alkyle en C₁-C₆, un radical alcoxy en C₁-C₆, un radical alkylsulfonyle en C₁-C₆ (-SO₂-alkyl), un radical alkylsulfonate en C₁-C₆ (-SO₃-alkyl), un radical cyano, un radical trifluorométhyle, un radical alkylcarbonyle en C₁-C₆, un radical trifluorométhylsulfonyle (-SO₂-CF₃), un radical trifluorométhylcarbonyle, un radical phénylsulfonyle (-SO₂-Ph), un radical phénylsulfonate (-SO₃-Ph) un radical phénylcarbonyle, un radical nitro, un radical alcoxycarbonyle en C₁-C₆, un radical phosphonyle (-PO(OH)₂), un radical phosphonylalkyle(C₁-C₆) (-alkyl-PO(OH)₂), un radical hydroxyle, un radical amino, un radical dialkyl(C₁-C₆)amino, un radical (hydroxyalkyl(C₁-C₆))amino, un radical di(hydroxyalkyl(C₁-C₆))amino, un radical (aminoalkyl(C₁-C₆))amino, un radical di(aminoalkyl(C₁-C₆))amino, un radical (hydroxyalkyl(C₁-C-₆))(alkyl(C₁-C₆))amino, un radical (aminoatkyl(C₁-C₆))(alkyl(C₁-C₆))amino, un radical (aminoalkyl(C₁-C₆))(hydroxyalkyl(C₁-C₆)amino, un radical hydroxyalkyle(C₁-C₆), un radical aminoalkyle(C₁-C₆), un radical (alkyl(C₁-C₆))aminoalkyle(C₁-C₆), un radical di(alkyl(C₁-C₆))aminoalkyle(C₁-C₆), un radical (hydroxyalkyl(C₁-C₆))aminoalkyle(C₁-C₆), un radical di(hydroxyalkyl(C₁-C₆))aminoalkyle(C₁-C₆), un radical (aminoalkyl(C₁-C₆))aminoalkyle(C₁-C₆), un radical di(aminoalkyl(C₁-C₆))aminoalkyle(C₁-C₆), un radical (alkyl(C₁-C₆))(hydroxyalkyl(C₁-C₆))aminoalkyle(C₁-C₆), un radical (aminoalkyl(C₁-C₆))(alkyl(C₁-C₆))amino, un radical (hydroxyalkyl(C₁-C₆))(alkyl(C₁-C₆))aminoalkyle(C₁-C₆), un radical phénylalkyle(C₁-C₆) éventuellement substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un groupement cationique du type ammonium quaternaire, un radical alkyle en C₁-C₆ substitué par un groupement cationique du type ammonium quaternaire, un radical carboxyle, un radical alkyle(C₁-C₆) substitué par un radical carboxyle, un radical thiol, un radical thioalkyle(C₁-C₆), un radical sulfonate (-SO₃⁻), un radical alkyle(C₁-C₆) substitué par un radical sulfonate, un radical alkyl(C₁-C₆)carbonylalkyle(C₁-C₆), un radical di(halogénoalkyl(C₁-C₆))amino, un radical acétamido, un radical aryloxy, un radical aryloxyalkyle(C₁-C₆), un radical éthényle (-CH=CH₂), un radical éthénylcarbonyle (-CO-CH=CH₂) ; deux groupements adjacents pouvant former un cycle aromatique ou hétéroaromatique, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, ou un cycle de type -O-CₘH₂ₘ-O- où m est un entier égal à 1 ou 2. De préférence, A est non substitué ou substitué par un ou plusieurs groupements choisis parmi un radical alkyl(C₁-C₆)sulfonyle ; un groupement pyridinium ou imidazolium, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ; un groupement trialkyl(C₁-C₆)ammonium ; un radical sulfonate. A titre d'exemple, A peut être substitué par un radical méthylsulfonyle ; un groupement 1-méthyl-pyridinium ; un groupement imidazolium ; un groupement triméthylammonium ; un radical sulfonate.

Selon un mode de réalisation particulier de l'invention, X est choisi parmi un groupement CR₁R₂.

Selon un mode de réalisation particulier de l'invention, R₁ et R₂ sont choisis parmi un radical alkyle en C₁-C₆. A titre d'exemple, R₁ et R₂ peuvent être un radical méthyle ; un radical éthyle.

Selon un mode de réalisation particulier de l'invention, R₃ est choisi parmi un atome d'hydrogène.

Selon un mode de réalisation particulier de l'invention, W est choisi parmi un groupement CR₄.

Selon un mode de réalisation particulier de l'invention, R₄ est choisi parmi un atome d'hydrogène.

Selon un mode de réalisation particulier de l'invention, Y est choisi parmi un atome d'oxygène ou un atome de soufre.

Selon un mode de réalisation particulier de l'invention, Z est choisi parmi un groupement -CₚH₂ₚ-, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle. A titre d'exemple, Z peut être un groupement -C₂H₄-.

Selon un mode de réalisation particulier de l'invention, n est égal à 1 ou 2.

Selon un mode de réalisation particulier de l'invention, B est un noyau benzénique, carbazolique ou indolique.

Selon un mode de réalisation particulier de l'invention, B est non substitué ou substitué par un ou plusieurs groupements pouvant être choisis parmi un radical halogéno, un radical alkyle en C₁-C₆, un radical alcoxy en C₁-C₆, un radical cyano, un radical trifluorométhyle, un radical alkylcarbonyle en C₁-C₆, un radical trifluorométhylsulfonyle, un radical trifluorométhylcarbonyle, un radical phénylsulfonyle, un radical phénylcarbonyle, un radical phényle non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un radical acylamino, un radical hydroxyle, un radical amino, un radical di(alkyl(C₁-C₆))amino, un radical hydroxyalkyl(C₁-C₆)amino, un radical di(hydroxyalkyl(C₁-C₆))amino, un radical (aminoalkyl(C₁-C₆))amino, un radical di(aminoalkyl(C₁-C₆))amino, un radical (alkyl(C₁-C₆))(hydroxyalkyl(C₁-C-₆))amino, un radical (aminoalkyl(C₁-C₆))(alkyl(C₁-C₆))amino, un radical (aminoalkyl(C₁-C₆))(hydroxyalkyl(C₁-C₆))amino, un radical hydroxyalkyle(C₁-C₆), un radical aminoalkyle(C₁-C₆), un radical alkyl(C₁-C₆)aminoalkyle(C₁-C₆), un radical di(alkyl(C₁-C₆))aminoalkyle(C₁-C₆), un radical (hydroxyalkyl(C₁-C₆))àminoalkyle(C₁-C₆), un radical di(hydroxyalkyl(C₁-C₆))aminoalkyle(C₁-C₆), un radical aminoalkyl(C₁-C₆)aminoalkyle(C₁-C₆), un radical di(aminoalkyl(C₁-C₆))aminoalkyle(C₁-C₆), un radical (alkyl(C₁-C₆))(hydroxyalkyl(C₁-C₆))aminoalkyle(C₁-C₆), un radical (alkyl(C₁-C₆))(aminoalkyl(C₁-C₆))amino, un radical (hydroxyalkyl(C₁-C₆))(alkyl(C₁-C₆))aminoalkyle(C₁-C₆), un radical phénylalkyle(C₁-C₆) non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, un groupement cationique du type ammonium quaternaire, un radical alkyle(C₁-C₆) substitué par un groupement cationique du type ammonium quaternaire, un radical carboxyle, un radical alkyle(C₁-C₆) substitué par un radical carboxyle, un radical thiol, un radical thioalkyle(C₁-C₆), un radical sulfonate, un radical alkyle(C₁-C₆) substitué par un radical sulfonate, un radical alkyl(C₁-C₆)carbonylalkyle(C₁-C₆), un radical di(halogénoalkyl(C₁-C₆))amino, un radical acétamido, un radical aryloxy, un radical aryloxyalkyle(C₁-C₆), un radical éthényle, un radical éthénylcarbonyle, un groupement NR₆R₇, R₆ et R₇ pouvant former ensemble avec l'atome d'azote auquel ils sont attachés un cycle en C₅, C₆ ou C₇ non aromatique, éventuellement interrompu par un ou plusieurs hétéroatomes tels qu'un atome d'azote, d'oxygène ou de soufre, une chaîne alkylène pouvant contenir un atome d'oxygène ou de soufre et pouvant être terminée par un groupement cyano, alkylsulfonyle en C₁-C₆ ou alkylcarbonyle en C₁-C₆ ; deux groupements adjacents de B pouvant former un cycle aromatique ou hétéroaromatique, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle, ou un cycle de type -O-CᵣH₂ᵣ-O- où r représente un entier égal à 1 ou 2. De préférence, B est non substitué ou substitué par un ou plusieurs groupements choisis parmi un radical hydroxyle ; un radical amino ; un radical di(alkyl(C₁-C₆))amino ; un radical alkyle en C₁-C₆; un radical acétamido ; un groupement pyridinium ; un groupement trialkyl(C₁-C₆)ammonium. A titre d'exemple, B peut être substitué par un radical hydroxyle ; un radical amino ; un radical diméthylamino ; un radical éthyle ; un radical acétamido ; un groupement pyridinium ; un groupement triméthylammonium.

Les groupements cationiques du type ammonium quaternaire peuvent par exemple être choisis parmi les groupements trialkylammonium, oxazolium, thiazolium, imidazolium, pyrazolium, pyridinium, pyrrolium, triazolium, isooxazolium, isothiazolium, pyrimidinium, pyrazinium, triazinium, pyridazinium, indolium, quinolinium ou isoquinolimiun, substitué ou non substitué, et peuvent être reliés au noyau A ou au noyau B par n'importe lequel de leurs atomes de carbone non substitués.

A titre d'exemples de composés de formule (I) pour lesquels Y est un atome d'oxygène, on peut citer l'acide 9a-[2-[4-(diméthylamino)phényl]-1,3-butadiényl]-2,3,9,9a-tétrahydro-9,9-diméthyl oxazolo[3,2-a]indole-7-carboxylique ; l'acide [9a-[2-[4-(diméthylamino)phényl]éthènyl]-2,3,9,9a-tétrahydro-9,9-diméthyloxazolo[3,2-a]indol-7-yl] phosphonique ; la 4-[2-(9,9-diéthyl-2,3-dihydro-7-méthoxyoxazolo[3,2-a]indol-9a(9H)-yl)éthènyl]-N,N-diéthyl benzenamine ; l'acide [3-[9a-[2-[4-(diméthylamino)phényl]éthènyl]-2,3,9,9a-tétrahydro-9,9-diméthyl oxazolo[3,2-a]indol-7-yl]propyl] phosphonique ; la 4-[2-(2,3-dihydro-9,9-diméthyl oxazolo[3,2-a]indol-9a(9H)-yl)éthènyl]-N-méthyl-N-phényl benzènamine ; la 4-[2-(2,3-dihydro-9,9-diméthyloxazolo[3,2-a]indol-9a(9H)-yl)éthènyl]-3-éthoxy-N,N-diéthyl benzènamine ; la 4-[2-(2,3-dihydro-9,9-diméthyloxazolo[3,2-a]indol-9a(9H)-yl)éthènyl]-N-éthyl-N-(2-méthylpropyl) benzènamine ; la 4-[2-(2,3-dihydrooxazolo[3,2-a]indol-9a(9H)-yl)éthènyl]-N,N-diméthyl benzènamine ; la 4-[4-(2,3-dihydro-7,9,9-triméthyloxazolo[3,2-a]indol-9a(9H)-yl)-1,3-butadiènyl]-N,N-diméthyl benzènamine ; la 4-[4-(2,3-dihydro-9,9-diméthyl-7-nitrooxazolo[3,2-a]indol-9a(9H)-yl)-1,3-butadiènyl]-N,N-diméthyl benzenamine ; le 9a-[4-[4-(diméthylamino)phényl]-1,3-butadiènyl]-2,3,9,9a-tétrahydro-9,9-diméthyl oxazolo[3,2-a]indole-7-carbonitrile ; le 9a-[2-[4-(diméthylamino) phényl]éthènyl]-2,3,9,9a-tétrahydro-9,9-diméthyl oxazolo[3,2-a]indole-7-carbonitrile ; la 4-[2-(2,3-dihydro-9,9-diméthyloxazolo[3,2-a]indot-9a(9H)-yl)éthènyl]-N,N-diméthyl benzenamine ; l'acide 9a-[2-[4-(diméthylamino)phényl]éthènyl]-2,3,9,9a-tétrahydro-9,9-diméthyl-méthylester oxazolo[3,2-a]indole-7-sulfonique ; la N,N-bis(2-chloroéthyl)-4-[2-(2,3-dihydro-9,9-diméthyloxazolo[3,2-a]indol-9a(9H)-yl)éthènyl] benzènamine ; la 9a-[2-[4-(diméthylamino)phényl]éthènyl]-9,9a-dihydro-9,9-diméthyl-7-(octylsulfonyl) oxazolo[3,2-a]indol-2(3H)-one ; la 9a-[2-[4-(diméthylamino) phényl]éthènyl]-9,9a-dihydro-9,9-diméthyl-7-(phénylsulfonyl) oxazolo [3,2-a]indol-2(3H)-one ; la 9a-[2-[4-(diméthylamino)phényl]-1-méthyléthènyl]-9,9a-dihydro-9,9-diméthyl-7-(méthylsulfonyl) oxazolo[3,2-a]indol-2(3H)-one ; la 9a-[2-[4-(diméthylamino) phényl]éthènyl]-9,9a-dihydro-9,9-diméthyl-7-(méthylsulfonyl) oxazolo [3,2-a]indol-2(3H)-one ; la 4-[2-[2,3-dihydro-9,9-diméthyl-7-(méthylsulfonyl) oxazolo[3,2-a]indol-9a(9H)-yl]éthènyl]-N,N-diméthyl benzènamine ; la 4-[2-[9-(éthoxyméthyl)-2,3-dihydro-9-méthyl-7-(méthylsulfonyl)oxazolo[3,2-a]indol-9a(9H)-yl]éthènyl]-N,N-diméthyl benzenamine ; la 4-[2-[2,3-dihydro-2,9,9-triméthyl-7-(méthylsulfonyl)oxazolo[3,2-a]indol-9a(9H)-yl]éthènyl]-N,N-diméthyl benzènamine ; la 4-[2-[2,3-dihydro-9,9-diméthyl-7-(méthylsulfonyl)oxazolo[3,2-a]indol-9a(9H)-yl]-1-propényl]-N,N-diméthyl benzènamine ; la N,N-dibutyl-4-[2-[2,3-dihydro-9,9-diméthyl-7-(méthylsulfonyl)oxazolo [3,2-a]indol-9a(9H)-yl]éthènyl] benzènamine ; 4-[2-[2,3-dihydro-9,9-diméthyl-7-(phénylsulfonyl)oxazolo[3,2-a]indol-9a(9H)-yl]éthènyl]-N,N-diméthyle ; la 4-[2-[2,3-dihydro-9,9-diméthyl-7-(octylsulfonyl)oxazolo[3,2-a]indol-9a(9H)-yl]éthènyl]-N,N-diméthylbenzènamine ; le N-[4-[2-[7-(butylsulfonyl)-2,3-dihydro-9,9-diméthyl oxazolo [3,2-a]indol-9a(9H)-yl]éthènyl]phényl] acétamide ; la 4-[2-[7-(butylsulfonyl)-2,3-dihydro-9,9-diméthyloxazolo[3,2-a]indol-9a(9H)-yl]éthènyl]-N,N-diméthyl benzènamine ; la 4-[4-[2,3-dihydro-9,9-diméthyl-7-(méthylsulfonyl)oxazolo[3,2-a]indol-9a(9H)-yl]-1,3-butadiènyl]-N,N-diméthyl benzènamine ; le 9a-[2-[4-(diméthylamino)phényl]éthènyl]-2,3,9,9a-tétrahydro-9-méthyl oxazolo[3,2-a]indole-9-éthanol ; la 4-[2-(9,9-diéthyl-2,3-dihydrooxazolo[3,2-a]indol-9a(9H)-yl)éthènyl]-N,N-diméthyl benzènamirie ; la 4-[2-[2,3-dihydro-9-méthyl-9-(2-phénoxyéthyl)oxazolo[3,2-a]indol-9a(9H)-yl]éthènyl]-N,N-diméthyl benzènamine ; la 4-[2-[9-(éthoxyméthyl)-2,3-dihydro-9-méthyloxazolo[3,2-a]indol-9a(9H)-yl]éthènyl]-N,N-diméthyl benzènamine ; la 4-[2-(11,11-diméthylbenz[e] oxazolo[3,2-a]indol-10a(11H)-yl)éthènyl]-N,N-diméthyl benzenamine ; la 4-[2-(7-méthoxy-9,9-diméthyloxazolo[3,2-a]indol-9a(9H)-yl)éthènyl]-N,N-diméthyl benzènamine ; la 4-[2-(9,9-diméthyloxazolo[3,2-a]indol-9a(9H)-yl)éthènyl]-N,N-diméthyl benzènamine ; la 9a-[2-[4-(diméthylamino)phényl]éthènyl]-3-éthyl-9,9a-dihydro-9,9-diméthyloxazolo[3,2-a]indol-2(3H)-one ; la 7-chloro-9a-[2-[4-(diméthylamino)phényl] éthènyl]-9,9a-dihydro-3,9,9-triméthyl oxazolo[3,2-a]indol-2(3H)-one ; la 9a-[2-[4-(dibutylamino)phényl]éthènyl]-9,9a-dihydro-9,9-diméthyl oxazolo[3,2-a]indol-2(3H)-one ; la 9a-[2-[4-(diméthylamino)phényl]éthènyl]-9,9a-dihydro-9-(2-hydroxyéthyl)-9-méthyl oxazolo[3,2-a]indol-2(3H)-one ; la 9a-[2-[4-(diméthylamino)phényl]-1-propényl]-9,9a-dihydro-9,9-diméthyl oxazolo[3,2-a]indol-2(3H)-one ; la 9,9a-dihydro-7,9,9-triméthyl-9a-[2-(4-nitrophényl)éthènyl] oxazolo[3,2-a]indol-2(3H)-one , le N-[4-[2-(2,3-dihydro-9,9-diméthyl-2-oxooxazolo[3,2-a]indol-9a(9H)-yl)éthènyl]phényl] acetamide ; la 9a-[2-[4-(diméthylamino)phényl]éthènyl]-9,9a-dihydro-6-méthoxy-9,9-diméthyl oxazolo[3,2-a]indol-2(3H)-one ; l'acide 9a-[2-[4-(diméthylamino)phényl]éthènyl]-2,3,9,9a-tétrahydro-9,9-diméthyl-2-oxo-éthyl ester oxazolo[3,2-a]indole-7-carboxylique ; la 9a-[2-[4-(diméthylamino)phényl]éthènyl]-9,9a-dihydro-7,9,9-triméthyl oxazolo[3,2-a]indol-2(3H)-one ; la 10a-[2-[4-(diméthylamino)phényl]éthènyl]-10a,11-dihydro-11,11-diméthyl benz[e]oxazolo[3,2-alindol-9(8H)-one ; la 9,9a-dihydro-9,9-diméthyl-9a-[2-(4-nitrophényl)éthènyl] oxazolo[3,2-a]indol-2(3H)-one ; la 7-chloro-9a-[2-[4-(diméthylamino)phényl]éthènyl]-9,9a-dihydro-9,9-diméthyl oxazolo[3,2-a]indol-2(3H)-one ; la 9a-[2-[4-(diméthylamino)phényl]éthènyl]-9,9a-dihydro-9,9-diméthyl oxazolo[3,2-a]indol-2(3H)-one ; la 4-[2-(7-chloro-2,3-dihydro-9,9-diméthyloxazolo[3,2-a]indol-9a(9H)-yl)éthènyl]-N,N-diméthyl benzènamine ; la 4-[2-(2,3-dihydro-9,9-diméthyloxazolo[3,2-a]indol-9a(9H)-yl)éthènyl]-N,N-diméthyl benzènamine ; la 4-[2-(7-chloro-2,3-dihydro-2,9,9-triméthyloxazolo[3,2-a]indol-9a(9H)-yl)éthènyl]-N,N-diméthyl benzènamine ; la 4-[2-(2,3-dihydro-9,9-diméthyloxazolo[3,2-a]indol-9a(9H)-yl)-1-méthyléthènyl]-N,N-diméthyl benzènamine ; la 4-[2-(2,3-dihydro-9,9-diméthyloxazolo[3,2-a]indol-9a(9H)-yl)éthènyl]-N,N-diéthyl benzènamine ; le 2,3,9,9a-tétrahydro-7-méthoxy-9,9-diméthyl-9a-[2-(4-nitrophényl)éthènyl] oxazolo[3,2-a]indole ; la 4-[2-(2,3-dihydro-9,9-diméthyl-7-nitrooxazolo[3,2-a]indol-9a(9H)-yl)éthènyl]-N,N-diméthyl benzènamine; la N,N-dibutyl-4-[2-(2,3-dihydro-9,9-diméthyloxazolo[3,2-a]indol-9a(9H)-yl)éthènyl] benzènamine ; le 2,3,9,9a-tétrahydro-9,9-diméthyl-7-nitro-9a-[2-(4-nitrophényl)éthènyl] oxazolo[3,2-a]indole ; le 7-chloro-2,3,9,9a-tétrahydro-9,9-diméthyl-9a-[2-(4-nitrophényl)éthènyl] oxazolo[3,2-a]indole ; la 4-[2-(2,3-dihydro-7-iodo-9,9-diméthyloxazolo[3,2-a]indol-9a(9H)-yl)éthènyl]-N,N-diméthyl benzènamine ; la 4-[2-(2,3-dihydro-5-méthoxy-9,9-diméthyloxazolo[3,2-a]indol-9a(9H)-yl)éthènyl]-N,N-diméthyl benzènamine ; la 4-[2-(2,3-dihydro-7-méthoxy-9,9-diméthyloxazolo[3,2-a]indol-9a(9H)-yl)éthènyl]-N,N-diméthyl benzènamine ; la 4-[4-(2,3-dihydro-9,9-diméthyloxazolo[3,2-a]indol-9a(9H)-yl)-1,3-butadiènyl]-N,N-diéthyl benzènamine ; la 4-[4-(7-chloro-2,3-dihydro-9,9-diméthyloxazolo[3,2-a]indol-9a(9H)-yl)-1,3-butadiènyl]-N,N-diméthyl benzènamine ; la 4-[4-(2,3-dihydro-9,9-diméthyloxazolo[3,2-a]indol-9a(9H)-yl)-1,3-butadiènyl]-N,N-diméthyl benzènamine ; la 4-[4-(2,3-dihydro-7-méthoxy-9,9-diméthyloxazolo[3,2-a]indol-9a(9H)-yl)-1,3-butadiènyl]-N,N-diméthyl benzènamine ; le 2,3,9,9a-tétrahydro-7,9,9-triméthyl-9a-[2-(4-nitrophényl)éthènyl] oxazolo[3,2-a]indole ; le N-[4-[2-(2,3-dihydro-9,9-diméthyloxazolo[3,2-a]indol-9a(9H)-yl)éthènyl]phényl] acétamide ; la 4-[2-(2,3-dihydro-6-méthoxy-9,9-diméthyloxazolo[3,2-a]indol-9a(9H)-yl)éthènyl]-N,N-diméthyl benzènamine; la 4-[2-(8,9-dihydro-11,11-diméthylbenz[e]oxazolo[3,2-a]indol-10a(11H)-yl)éthènyl]-N,N-diméthyl benzènamine ; la 4-[2-(2,3-dihydro-7,9,9-triméthyloxazolo[3,2-a]indol-9a(9H)-yl)éthènyl]-N,N-diméthyl benzènamine ; le 2,3,9,9a-tétrahydro-9,9-diméthyl-9a-[2-(4-nitrophényl)éthènyl] oxazolo[3,2-a]indole ; la 9,9a-dihydro-9,9-diméthyl-9a-[2-(9-méthyl-9H-carbazol-3-yl)éthènyl]-7-(méthylsulfonyl) oxazolo[3,2-a]indol-2(3H)-one ; le 9a-[2-(9-hexyl-9H-carbazol-3-yl)éthènyl]-2,3,9,9a-tétrahydro-9,9-diméthyl-7-(phénylsulfonyl) oxazolo[3,2-a]indole ; le 2,3,9,9a-tétrahydro-9,9-diméthyl-7-(méthylsulfonyl)-9a-[2-(9-octyl-9H-carbazol-3-yl)éthènyl] oxazolo[3,2-a]indole ; l'acide 9a-[2-(9-butyl-6-méthoxy-9H-carbazol-3-yl)éthènyl]-2,3,9,9a-tétrahydro-9,9-diméthyl-, éthyl ester oxazolo[3,2-a]indole-7-carboxylique ; l'acide 9a-[2-(9-éthyl-9H-carbazol-3-yl)éthènyl]-2,3,9,9a-tétrahydro-9,9-diméthyl-, méthyl ester oxazolo[3,2-a]indole-7-sulfonique ; le 3-chloro-6-[2-[2,3-dihydro-9,9-diméthyl-7-(méthylsulfonyl)oxazolo[3,2-a]indol-9a(9H)-yl]éthènyl]-9-octyl 9H-carbazole ; le 3-[2-[2,3-dihydro-9,9-diméthyl-7-(méthylsulfonyl)oxazolo[3,2-a]indol-9a(9H)-yl]éthènyl]-9-méthyl 9H-carbazole ; le 3-[2-[9-(2-éthoxyéthyl)-2,3-dihydro-9-méthyl-7-(méthyl sulfonyl)oxazolo[3,2-a]indol-9a(9H)-yl]éthènyl] 9H-carbazole ; l'acide 9a-[2-(9-hexyl-9H-carbazol-3-yl)éthènyl]-2,3,9,9a-tétrahydro-9-(2-hydroxyéthyl)-9-méthyl-, éthyl ester oxazolo[3,2-a]indole-7-carboxylique ; l'acide 2,3,9,9a-tétrahydro-9,9-diméthyl-9a-[2-(9-octyl-9H-carbazol-3-yl)éthènyl]-, éthyl ester oxazolo[3,2-a]indole-7-carboxylique ; l'acide 9a-[2-(9-butyl-9H-carbazol-3-yl)éthènyl]-2,3,9,9a-tétrahydro-9,9-diméthyl-, éthyl ester oxazolo[3,2-a]indole-7-carboxylique ; l'acide 2,3,9,9a-tétrahydro-9,9-diméthyl-9a-[2-(9-méthyl-9H-carbazol-3-yl)éthènyl]-, éthyl ester oxazolo[3,2-a]indole-7-carboxylique ; la 9a-[2-(9-butyl-6-éthoxy-9H-carbazol-3-yl)éthènyl]-2,3,9,9a-tétrahydro-N,N,9,9-tétraméthyl oxazolo[3,2-a]indol-7-amine ; le 2,3,9,9a-tétrahydro-9-méthyl-9a-[2-(9-méthyl-9H-carbazol-3-yl)éthènyl] oxazolo[3,2-a]indole-9-éthanol ; la 9a-[2-(9-butyl-9H-carbazol-3-yl)éthènyl]-2,3,9,9a-tétrahydro-N,N,9,9-tétraméthyl oxazolo[3,2-a]indol-7-amine ; le 3-[2-(2,3-dihydro-6-méthoxy-9,9-diméthyloxazolo[3,2-a]indol-9a(9H)-yl)éthènyl]-9-méthyl 9H-carbazole ; le 3-[2-(2,3-dihydro-9,9-diméthyloxazolo[3,2-a]indol-9a(9H)-yl)-1-propényl]-9-méthyl 9H-carbazole ; le 3-[2-(7-chloro-2,3-dihydro-9,9-diméthyloxazolo[3,2-a]indol-9a(9H)-yl)éthènyl]-9-méthyl 9H-carbazole ; le 3-bromo-6-[2-(2,3-dihydro-9,9-diméthyloxazolo[3,2-a]indol-9a(9H)-yl)éthènyl]-9-éthyl 9H-carbazole ; le 3-[2-(2,3-dihydro-9,9-diméthyloxazolo[3,2-a]indol-9a(9H)-yl)éthènyl]-9-méthyl 9H-carbazole.

De préférence, le ou les composés de formule (I) pour lesquels Y représente un atome d'oxygène, sont choisis parmi les composés présentés dans le tableau ci-dessous :

| | |
|---|---|
| 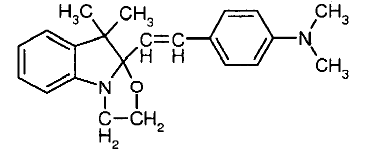 | Colorant 1 |
| 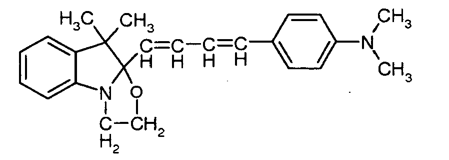 | Colorant 2 |
| 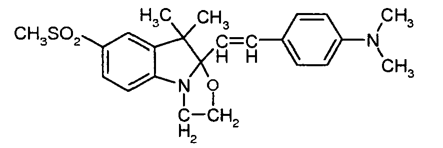 | Colorant 3 |
| 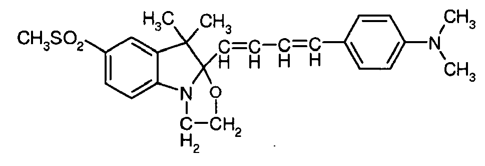 | Colorant 4 |
| 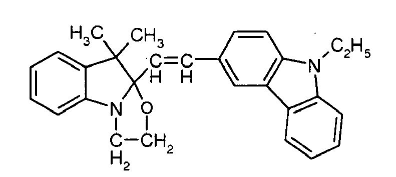 | Colorant 5 |
| 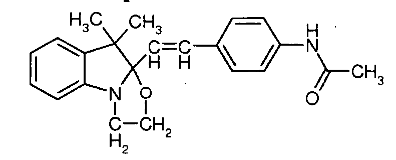 | Colorant 6 |
| 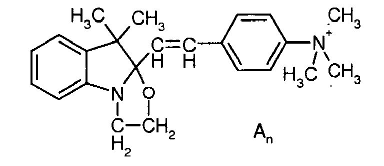 | Colorant 7 |
| 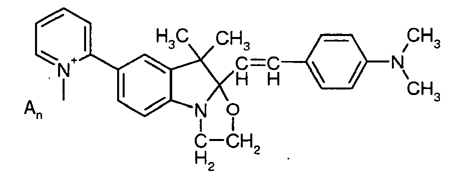 | Colorant 8 |
| 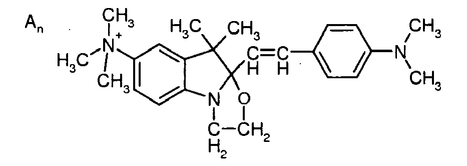 | Colorant 9 |
| 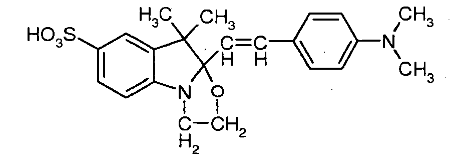 | Colorant 10 |
| 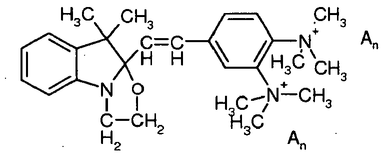 | Colorant 11 |

Dans le tableau ci-dessus, An représente un contre-ion tel que défini précédemment.

De préférence, les composés sont choisis parmi les colorants 1 à 6 et 8 ci-dessus.

A titre d'exemples de composés de formule (I) pour lesquels Y est un atome de soufre, on peut citer le 3,3-diméthyl-2-(p-diméthylaminostyryl)indolino[1,2-b]thiazoline ; le 3,3,5-triméthyl-2-(p-diméthylaminostyryl)indolino[1,2-b]thiazoline ; le 3,3,5-triméthyl-2-(p-chlorostyryl)indolino[1,2-b]thiazoline ; le 3,3,5-triméthyl-2-[2-(thiényl)vinyl] indolino[1,2-b]thiazoline ; le 3,3-diméthyl-5-méthoxy-2-[2-(9-éthylcarbazolyl)vinyl] indolino[1,2-b]thiazoline ; le 3,3-diméthyl-5-carboéthoxy-2-(p-diméthylaminostyryl) indolino[1,2-b]thiazoline ; le 3,3-diméthyl-5-chloro-2-[2-(benzothiazolyl)vinyl)indolino [1,2-b]thiazoline ; le 3,3-diméthyl-5-méthoxy-2-(p-diméthylaminostyryl)indolino[1,2-b]thiazoline ; le 3,3-diméthyl-5-carboéthoxy-2-(3,4-méthylènedioxystyryl)indolino[1,2-b]thiazoline ; le 3,3-diméthyl-5-chloro-2-(p-méthylstyryl)indolino[1 ,2-b]thiazoline ; le 3,3-diméthyl-5-chloro-2-(p-méthoxystyryl)indolino[1,2-b]thiazoline; le 3,3,5-triméthyl-2-(p-acétylaminostyryl)indolino[1,2-b]thiazoline ; le 3,3-diméthyl-5-méthoxy-2-(3-hydroxy-4-méthoxystyryl)indolino[1,2-b]thiazoline ; le 3,3-diméthyl-5-carboéthoxy-2-(o-cyano styryl)indolino[1,2-b]thiazoline ; le 3,3-diméthyl-5-chloro-2-(p-diméthylaminostyryl) indolino[1,2-b]thiazoline ; le 3,3-diméthyl-5-méthylsulfonyl-2-(p-diméthylaminostyryl) indolino[1,2-b]thiazoline ; le 3,3-diméthyl-5-phénylsulfonyl-2-(p-diméthylaminostyryl) indolino[1,2-b]thiazoline ; le 3,3-diméthyl-5-éthoxycarboxy-2-(p-diméthylaminostyryl) indolino[1,2-b]oxazoline.

Les composés de formule (I) utiles présents dans la composition de l'invention peuvent par exemple être préparés selon les modes de préparation tels que décrits dans les brevets FR 2 285 439 et US 4 380 629. Ces modes de préparation peuvent être adaptés aux composés de formule (I) cationiques en ajoutant une étape de quaternisation.

A titre d'exemple de synthèse de composé de formule (I) avec Y représentant un atome d'oxygène, on peut réaliser la synthèse du colorant 8 selon le schéma réactionnel suivant :

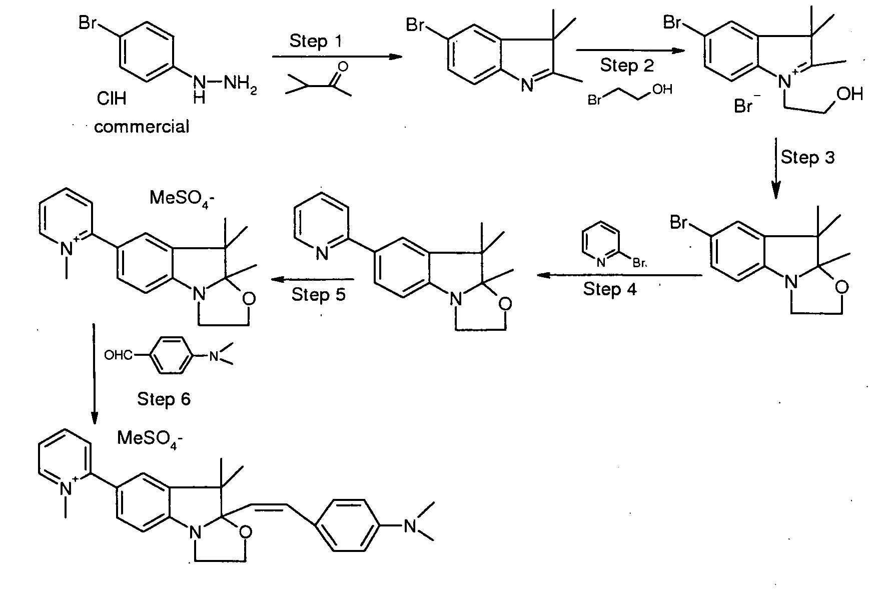

A titre d'exemple de synthèse de composé de formule (I) avec Y représentant un atome de soufre, on peut réaliser la synthèse comme suit :

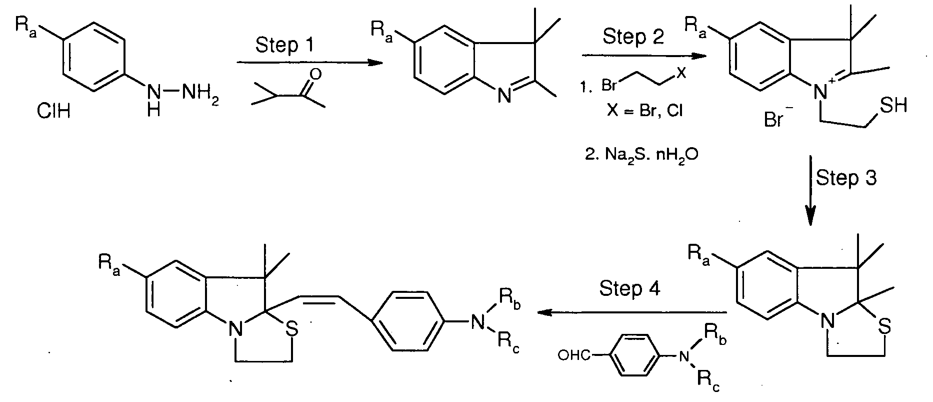

Le ou les composés choisis parmi les composés de formule (I) et leurs sels d'addition représentent en général de 0,0001 à 30 % en poids par rapport au poids total de la composition, plus particulièrement de 0,001 à 10 % en poids par rapport au poids total de la composition, et de préférence de 0,01 à 5 % en poids par rapport au poids total de la composition.

D'une manière générale, les sels d'addition des composés de formule (I) utilisables dans le cadre de l'invention sont notamment choisis parmi les sels d'addition avec un acide tels que les chlorhydrates, les bromhydrates, les sulfates, les méthosulfates, les gluconates, les citrates, les succinates, les tartrates, les lactates, les tosylates, les benzènesulfonates, les phosphates et les acétates et les sels d'addition avec une base telles que la soude, la potasse, l'ammoniaque, les amines dont les alcanolamines.

Comme indiqué auparavant, la composition selon l'invention comprend au moins un composé thiolé, différent des composés de formule (I), comprenant au moins une fonction thiol (SH) et un groupement comprenant de 1 à 20 atomes de carbone, saturé ou non, ledit groupement étant éventuellement interrompu par un ou plusieurs groupes non adjacents (séparés par au moins un atome de carbone) choisis parmi -O-, -S-, -S-S-, amino (-NR-), carbonyle (-CO-), oxycarbonyle (-O-CO-), aminocarbonyle (-NR-CO-), noyau aromatique ou hétéroaromatique, les groupements amino étant ou non substitués par un ou deux radicaux alkyle en C₁-C₆ ; à la condition que l'atome de soufre de la fonction thiol soit rattaché audit groupement au moyen d'un atome de carbone.

Par ailleurs le groupement comprenant de 1 à 20 atomes de carbone peut éventuellement être substitué par un ou plusieurs hydroxyle, alcoxy en C₁-C₆, hydroxycarbonyle, alcoxy(C₁-C₆)carbonyle, amino, aminocarbonyle, alkylcarbonylamino, dans lesquels la fonction amino est substituée ou non par un ou deux radicaux alkyle en C₁-C₆.

De préférence le composé thiolé comprend une ou deux fonctions thiol (SH), de préférence une fonction thiol, et un groupement saturé comprenant de 2 à 10 atomes de carbone, ledit groupement étant éventuellement interrompu par un groupe choisi parmi les groupes carbonyle (-CO-), oxycarbonyle (-O-CO-) ; à la condition que l'atome de soufre de la fonction thiol soit rattaché audit groupement au moyen d'un atome de carbone.

Par ailleurs le groupement peut éventuellement être substitué par un ou plusieurs hydroxyle, alcoxy(C₁-C₆)carbonyle, amino substituée ou non par un ou deux radicaux alkyle en C₁-C₆.

Conformément à un mode de réalisation particulier de l'invention, le composé thiolé est choisi parmi l'acide thioglycolique, l'acide thiolactique, l'acide mercaptopropionique, la cystéamine, l'acide thiosuccinique, la cystéine, l'acétylcystéine, le glycéryl thioglycolate, la thioglycérine, leurs sels de métaux alcalins (sodium, potassium), alcalino-terreux (calcium) ou d'ammonium, ainsi que leurs mélanges.

Habituellement, la teneur en composé thiolé est comprise entre 0,001% et 30% en poids par rapport au poids total de la composition, plus particulièrement entre 0,01% et 15% en poids par rapport au poids total de la composition, de préférence entre 0,1% et 10% en poids par rapport au poids total de la composition, et de manière encore plus préférée entre 0,5 et 5 % en poids par rapport au poids total de la composition.

Il est à noter que le composé thiolé peut être mélangé à la composition comprenant le ou les composés de formule (I) et stocké ainsi. Selon une autre possibilité, le composé thiolé n'est mélangé à la composition comprenant le ou les composés de formule (I) qu'au moment de l'emploi de ladite composition.

La composition selon l'invention peut en outre comprendre un ou plusieurs colorants directs additionnels pouvant notamment être choisis parmi les colorants nitrés de la série benzénique, les colorants directs azoïques, les colorants directs méthiniques et les colorants naturels. Ces colorants directs peuvent être de nature non ionique, anionique ou cationique.

S'ils sont présents, la teneur en colorant(s) direct(s) additionnel(s) représente en général de 0,001 à 20 % en poids par rapport au poids total de la composition, de préférence de 0,01 à 10 % en poids par rapport au poids total de la composition.

La composition selon l'invention de même comprendre un ou plusieurs colorants d'oxydation choisis parmi les bases d'oxydation et les coupleurs classiquement utilisés en coloration d'oxydation.

Les bases d'oxydation peuvent être choisies parmi les para-phénylènediamines, les bis-phénylalkylènediamines, les para-aminophénols, les bis-para-aminophénols, les ortho-aminophénols, les ortho-phénylènediamines, les bases hétérocycliques et leurs sels d'addition.

Les coupleurs peuvent être choisis parmi les méta-phénylènediamines, les méta-aminophénols, les méta-diphénols, les coupleurs naphtaléniques, les coupleurs hétérocycliques et leurs sels d'addition.

S'ils sont présents, la teneur en colorant(s) d'oxydation représente en général de 0,001 à 20 % en poids par rapport au poids total de la composition, de préférence de 0,01 à 10 % en poids par rapport au poids total de la composition.

Conformément à un mode de réalisation préféré de l'invention, la composition tinctoriale ne comprend pas de colorant direct additionnel ni de colorant d'oxydation.

La composition peut également comprendre un ou plusieurs agents acidifiants et/ou un ou plusieurs agents alcalinisants habituellement utilisés en teinture des fibres kératiniques.

Parmi les agents acidifiants, on peut citer, à titre d'exemple, les acides minéraux comme par exemple l'acide chlorhydrique, l'acide nitrique, l'acide sulfurique ou les acides organiques comme par exemple les composés comprenant au moins une fonction acide carboxylique comme l'acide acétique, l'acide tartrique, l'acide citrique, l'acide lactique, l'acide succinique, l'acide malique, une fonction acide sulfonique, une fonction acide phosphonique ou une fonction acide phosphorique.

Parmi les agents alcalinisants on peut citer, à titre d'exemple :
- les acides aminés basiques ;
- les carbonates ou les bicarbonates de métaux alcalins ou alcalino-terreux ;
- les silicates ou les métasilicates ;
- les composés de formule (III) suivante :

   X(OH)ₙ (III)

   dans laquelle :
   - X représente un ion potassium, lithium, sodium, ammonium N⁺R₈R₉R₁₀R₁₁ avec R₈, R₉, R₁₀ et R₁₁, identiques ou différents, désignant un radical alkyle en C₂-C₄ lorsque n est égal à 1 ;
   - X représente un atome de magnésium ou de calcium lorsque n est égal à 2 ;
   et en particulier les hydroxyde de sodium ou de potassium ;
- les composés de formule (IV) suivante : 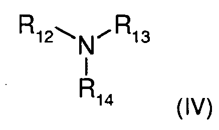 dans laquelle :
   - R₁₂ représente un atome d'hydrogène ; un radical alkyle en C₁-C₆, un radical monohydroxyalkyle en C₁-C₆ un radical polyhydroxyalkyle en C₂-C₆ ;
   - R₁₃ et R₁₄, identiques ou différents, représentent un atome d'hydrogène, un radical alkyle en C₁-C₆, un radical monohydroxyalkyle en C₁-C₆ ; un radical polyhydroxyalkyle en C₂-C₆ ;
   et en particulier l'ammoniaque et les alcanolamines telles que les mono-, di- et triéthanolamines ainsi que leurs dérivés ;
- les composés de formule (V) suivante : 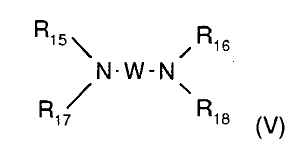 dans laquelle :
   - W est un reste propylène éventuellement substitué par un groupement hydroxyle ou un radical alkyle en C₁-C₄ ;
   - R₁₅ R₁₆, R₁₇ et R₁₈, identiques ou différents, représentent un atome d'hydrogène, un radical alkyle en C₁-C₄ ou hydroxyalkyle en C₁-C₄.

Au sens de la présente invention, on entend par "acide aminé basique", soit (i) un acide aminé présentant en plus de la fonction amine positionnée en α du groupement carboxyle, un groupement cationique (ou basique) supplémentaire; soit (ii) un acide aminé présentant une chaîne latérale (hydrophile) cationique (ou basique); soit (iii) un acide aminé portant une chaîne latérale constituée d'une base azotée. Ces définitions sont généralement connues et publiées dans les ouvrages de biochimie générale tels que J.H. WEIL (1983) pages 5 et suivantes, Lubert STRYER (1995) page 22, A. LEHNINGER (1993) pages 115-116, DE BOECK-WESMAEL (1994) pages 57-59.

Les acides aminés basiques conformes à l'invention sont choisis de préférence parmi ceux répondant à la formule (D) suivante : 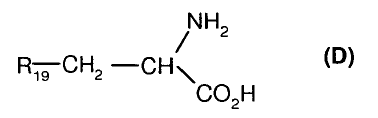 où R₁₉ désigne un groupe choisi parmi :

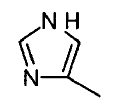

-(CH₂)₃NH₂

-(CH₂)₂NH₂

-(CH₂)₂NHCONH₂

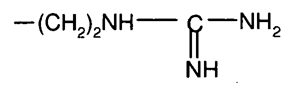

Parmi les composés de formule (D), on peut citer à titre d'exemple l'histidine, la lysine, l'ornithine, la citrulline, l'arginine.

Le milieu approprié pour la teinture, appelé aussi support de teinture, est généralement constitué par de l'eau ou par au moins un solvant organique ou par un mélange d'eau et d'au moins un solvant organique. A titre de solvant organique, on peut par exemple citer les cétones tels que l'acétone ; les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les polyols ou éthers de polyol tels que, par exemple, les éthers monométhylique, monoéthylique et monobutylique d'éthylèneglycol, le 2-butoxyéthanol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants sont généralement présents dans des proportions comprises entre 1 et 40 % en poids par rapport au poids total de la composition, et de préférence entre 5 et 30 % en poids par rapport au poids total de la composition.

La composition selon l'invention peut également renfermer divers adjuvants utilisés classiquement dans les compositions pour la teinture des cheveux, tels que des agents tensioactifs anioniques, cationiques, non ioniques, amphotères, zwitterioniques ou leurs mélanges, des polymères anioniques, cationiques, non ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des polymères cationiques ou amphotères, des cations, des silicones volatiles ou non volatiles, modifiées ou non modifiées, des chitosanes ou des dérivés de chitosane, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids total de la composition.

Bien entendu, l'homme de l'art veillera à choisir ce ou ces éventuels composés complémentaires de manière telle que les propriétés avantageuses attachées intrinsèquement à la composition de teinture utile dans le cadre de l'invention ne soient pas, ou substantiellement pas, altérées par la ou les adjonctions envisagées.

Selon une variante de l'invention, le pH de la composition est supérieur au pKa du composé de formule (I) et inférieur ou égal à 12. Avantageusement, le pH est compris entre 8 et 12. Dans le cadre de cette variante, le composé se trouve sous une forme substantiellement incolore.

La composition selon l'invention peut se présenter sous des formes diverses, telles que sous forme de liquides, de crèmes, de gels, ou sous toute autre forme appropriée pour réaliser une coloration des fibres kératiniques, et notamment des cheveux humains.

Le procédé de coloration des fibres kératiniques conforme à l'invention est un procédé dans lequel la composition qui vient d'être décrite est appliquée sur les fibres kératiniques, sèches ou humides, pendant un temps de pose suffisant, cette application étant éventuellement suivie d'un rinçage.

Les composés de formule (I) étant incolores, la couleur apportée par les composés de formule (I) est donc révélée par la suite. Ceci est réalisé en mettant en oeuvre des agents révélateurs tel que la lumière, un courant électrique, la chaleur, un agent acidifiant, un solvant, une radiation électromagnétique.

Lorsque la coloration est révélée par action de la chaleur, les fibres kératiniques peuvent être chauffées à l'aide d'un casque, d'un sèche cheveux ou d'un fer à friser ou à lisser.

En ce qui concerne la nature des agents acidifiants, des solvants, on pourra se reporter à ce qui a été indiqué auparavant dans la description.

De manière avantageuse, lorsque l'agent révélateur est un agent acidifiant, on préfère mettre en oeuvre au moins un acide organique comme par exemple les composés comprenant au moins une fonction acide carboxylique comme l'acide acétique, l'acide tartrique, l'acide citrique, l'acide lactique, l'acide succinique, l'acide malique.

Selon une première variante de l'invention, on effectue un post-traitement par action d'un agent révélateur tel que la lumière, un courant électrique, la chaleur, un agent acidifiant et/ou un solvant ou une radiation électromagnétique, ou d'une combinaison de plusieurs de ces agents révélateurs.

Selon une deuxième variante de l'invention, on effectue un pré-traitement par action d'un agent révélateur tel que les agents acidifiants et les solvants ou la combinaison d'un ou plusieurs de ces agents révélateurs.

Le temps de pose entre une étape de pré-traitement et l'application de la composition selon l'invention, ou bien entre l'application de ladite composition et une étape de post-traitement, peut être compris entre 5 minutes et 1 heure.

Il est de même possible de réduire ce temps de pose à zéro, ce qui revient à appliquer la composition selon l'invention immédiatement après l'application de la composition comprenant l'agent révélateur dans le cas d'une étape de pré-traitement, ou bien à une réalisation de l'étape de post-traitement immédiatement après l'application de la composition selon l'invention dans le cas d'une étape de post-traitement.

Selon un autre mode de réalisation particulier de l'invention, bien que non préféré, la composition selon l'invention est appliquée en présence d'un agent oxydant.

Selon une première possibilité, on applique simultanément l'agent oxydant et la composition comprenant le ou les composés de formule (I). Dans ce cas, on réalise une coloration et une décoloration simultanées des fibres kératiniques. Selon cette possibilité, l'agent oxydant est de préférence ajouté à la composition tinctoriale juste au moment de l'emploi.

Selon une seconde possibilité, on applique l'agent oxydant une fois que la composition comprenant le ou les composés de formule (I) est appliquée.

L'agent oxydant peut être choisi parmi le peroxyde d'hydrogène, le peroxyde d'urée, les bromates de métaux alcalins, les persels, les peracides et les enzymes de type oxydase.

Le temps de pose de la composition selon l'invention comprenant un agent oxydant ou non, ou d'application de l'agent révélateur, ou d'application de la composition comprenant un agent oxydant, est généralement compris entre 5 minutes et 1 heure, de préférence entre 15 minutes et 1 heure.

La température d'application de la composition selon l'invention comprenant un agent oxydant ou non, de l'agent révélateur et de la composition oxydante, est généralement fixée entre la température ambiante et 80°C, de préférence entre la température ambiante et 60 °C. A noter que dans le cas où l'agent révélateur est la chaleur, la température d'application est comprise entre 60 et 120°C.

La présente invention a également pour objet un dispositif à plusieurs compartiments permettant de mettre en oeuvre le procédé de coloration des fibres kératiniques conforme à l'invention.

Le dispositif à plusieurs compartiments de l'invention contient dans un premier compartiment une composition comprenant au moins un composé choisi parmi les composés de formule (I) et leurs sels d'addition et dans un deuxième compartiment au moins un composé thiolé, définis précédemment.

Selon un mode de réalisation particulier de l'invention, le dispositif à plusieurs compartiments de l'invention contient dans un troisième compartiment au moins un agent oxydant tel que défini précédemment.

Enfin, l'invention concerne également un procédé d'effaçage de la coloration obtenue au moyen de la composition selon l'invention.

Dans ce but, les fibres kératiniques colorées conformément à l'invention sont traitées avec une composition d'effaçage comprenant au moins un agent alcalinisant tel que décrit auparavant, avec une teneur telle que le pH des fibres traitées soit supérieur au pKa du ou des composés de formule (I) présents dans la composition à l'origine de la coloration.

La composition d'effaçage est appliquée sur les fibres sèches ou humides, avec habituellement un temps de pose compris entre 5 minutes et 1 heure, de préférence entre 5 et 30 minutes.

Classiquement, la température d'application de cette composition comprise entre la température ambiante et 80°C, de préférence entre la température ambiante et 60°C.

Les exemples qui suivent servent à illustrer l'invention sans toutefois présenter un caractère limitatif.

### Exemple 1

Les formulations suivantes ont été réalisées :

| | **A** **Comparative** | **B** **Invention** |
|---|---|---|
| Colorant (*) | 0,07% | 0,07% |
| Alcool benzylique | 8,4% | 8,4% |
| Ethanol | 25,2% | 25,2% |
| Monoéthanolamine | 4,2% | 4,2% |
| Acide thioglycolique | 0% | 1% |
| Eau distillée | qsp 100% | qsp 100% |

| | | |
|---|---|---|
| (*) Colorant : 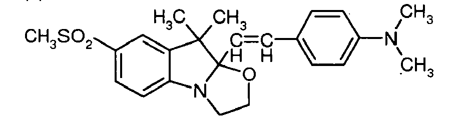 | | |

Chaque formulation est appliquée sur des mèches de cheveux 90% blancs naturels secs avec un temps de pose de 30 minutes à température ambiante.

La couleur est révélée ensuite par l'application d'une solution d'acide chlorhydrique à 0,5 N avec un rapport de bain de 1.

Les mèches sont ensuite rincées à l'eau claire et séchées à 60°C.

Les résultats colorimétriques (système CIEL*a*b*, composantes spéculaires inclues, angle 10°, D65) sont donnés ci-dessous dans le tableau 1.

| **Composition** | **L*** |
|---|---|
| **A comparative** | 54,3 |
| **B invention** | 32,2 |

On constate que l'intensité de la coloration obtenue en mettant en oeuvre la composition B selon l'invention comprenant 1 % d'acide thioglycolique, est significativement plus importante (valeur plus faible) que la puissance obtenue avec la composition comparative ne comprenant pas ce composé thiolé.

### Exemple 2

Les formulations selon l'invention suivantes ont été réalisées :

| | **C** | **D** | **E** | **F** | **G** | **H** |
|---|---|---|---|---|---|---|
| Colorant (*) | 0,07% | 0,07% | 0,07% | 0,07% | 0,07% | 0,07% |
| Alcool benzylique | 8,4% | 8,4% | 8,4% | 8,4% | 8,4% | 8,4% |
| Ethanol | 25,2% | 25,2% | 25,2% | 25,2% | 25,2% | 25,2% |
| Monoéthanolamine | 4,2% | 4,2% | 4,2% | 4,2% | 4,2% | 4,2% |
| Acide thioglycolique | 2% | - | - | - | - | - |
| Thioglycolate d'ammonium 71% | - | 3,34% | - | - | - | - |
| Dithioglycolate de diammonium | - | - | 9,77% | - | - | - |
| Thiolactate d'ammonium (70%) | - | - | - | 3,70% | - | - |
| Cystéine | - | - | - | - | 2,63% | - |
| N-acétyl-L-cystéine | - | - | - | - | - | 3,54% |
| Eau distillée | qsp 100% | qsp 100% | qsp 100% | qsp 100% | qsp 100% | qsp 100% |

Les formulations suivantes comparatives ont été réalisées :

| **Composé** | **I** | **J** | **K** | **L** |
|---|---|---|---|---|
| Colorant (*) | 0,07% | 0,07% | 0,07% | 0,07% |
| Alcool benzylique | 8,4% | 8,4% | 8,4% | 8,4% |
| Ethanol | 25,2% | 25,2% | 25,2% | 25,2% |
| Monoéthanolamine | 4,2% | 4,2% | 4,2% | 4,2% |
| Sodium thiosulfate pentahydrate (Na₂S₂O₃.5 H₂O) | 5,39% | - | - | - |
| Sulfite de sodium (Na₂SO₃) | - | 2,74% | - | - |
| Disulfite de sodium (métabisulfite de sodium pyrosulfite de sodium Na₂S₂O₅) | - | - | 3,54% | - |
| Sodium hydrogen sulfite | - | - | - | 2,28% |
| Eau distillée | qsp 100% | qsp 100% | qsp 100% | qsp 100% |

| | | | | |
|---|---|---|---|---|
| Colorant (*) : le colorant est le même que celui employé dans l'exemple 1. | | | | |

Les pourcentages des tableaux ci-dessus sont exprimés en poids. Les teneurs en composés thiolés correspondent à des teneurs identiques en moles %.

Les conditions d'application des compositions sont les mêmes que celles décrites dans l'exemple 1.

Les résultats colorimétriques (système CIEL*a*b*, composantes spéculaires inclues, angle 10°, D65) sont donnés dans le tableau 2 ci-dessous.

| | **L*** |
|---|---|
| Cheveux non colorés | 63,8 |
| Formulation C invention | 35,4 |
| Formulation D invention | 50,0 |
| Formulation E invention | 37,0 |
| Formulation F invention | 41,2 |
| Formulation G invention | 43,2 |
| Formulation H invention | 50,6 |
| Formulation I comparative | 59,0 |
| Formulation J comparative | 56,0 |
| Formulation K comparative | 63,5 |
| Formulation L comparative | 56,2 |

Plus la valeur de L* est faible, plus l'intensité de la coloration obtenue est importante.

Le tableau ci-dessus montre par conséquent, que les réducteurs de type thiolé provoquent une amélioration significative de l'intensité de la coloration obtenue entre les valeurs des coefficients L* des cheveux non colorés et des cheveux colorés avec des compositions comprenant des agents réducteurs hors invention, en particulier des agents réducteurs minéraux.

Par ailleurs, la composition appliquée sur les cheveux est incolore, ce qui fait que le procédé n'est pas salissant, ni les eaux de lavages et de rinçage des cheveux.

### Exemple 3

La composition suivante a été réalisée :

| **Composition tinctoriale** | **% poids** |
|---|---|
| Colorant (*) | 0,07% |
| Alcool benzylique | 5% |
| Ethanol | 25,2% |
| Monoéthanolamine | 2% |
| Thioglycolate d'ammionium (71 % MA) | 3,34 % |
| Hydroxyéthylcellulose | 1,5 |
| Eau distillée | qsp 100% |

| | |
|---|---|
| (*) Colorant : 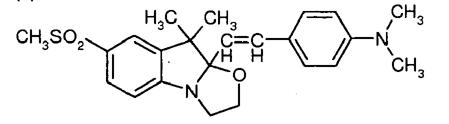 | |

Chaque formulation est appliquée sur des mèches de cheveux 90% blancs naturels secs, avec un rapport de bain de 5, un temps de pose de 30 minutes à température ambiante.

A l'issue de la coloration les cheveux sont rincés à l'eau claire puis essorés et traités au moyen d'une composition révélatrice dont la composition est résumée dans le tableau ci-dessous (les pourcentages sont exprimés en poids) :

| **Composition révélatrice** | **M** | **N** | **O** | **P** |
|---|---|---|---|---|
| Acide tartrique | 6% | - | - | - |
| Acide succinique | - | 5,9 | - | - |
| Acide DL malique | - | - | 6,7 | - |
| Acide citrique | - | - | - | 9,6 |
| NaOH qsp pH | 3 | 3 | 3 | 3,2 |
| Eau distillée | qsp 100% | qsp 100% | qsp 100% | qsp 100% |

Chaque composition est appliquée avec un rapport de bain de 1.

Les mèches sont ensuite rincées à l'eau claire et séchées à 60°C.

Les résultats colorimétriques (système CIEL*a*b*, composantes spéculaires inclues, angle 10°, D65) sont donnés ci-dessous :

| | **L*** |
|---|---|
| **Cheveux non colorés** | 63,8 |
| **Composition M** | 44,8 |
| **Composition N** | 39,2 |
| **Composition O** | 43,1 |
| **Composition P** | 38,9 |

## Revendications

1. Composition pour la coloration des fibres kératiniques, comprenant, dans un milieu approprié pour la teinture,
a) au moins un composé de formule (I) et ses sels d'addition : 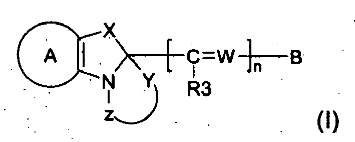 dans laquelle :
• A est un noyau aromatique ou hétéroaromatique, condensé ou non, comportant de 5 à 16 chaînons, non substitué ou substitué ;
• X représente un atome d'oxygène, un atome de soufre ou un groupement CR₁R₂ ;
• R₁ et R₂ représentent, indépendamment l'un de l'autre, un atome d'hydrogène, un radical alkyle en C₁-C₆, un radical hydroxyalkyle en C₁-C₆, un radical alcoxyalkyle en C₁-C₆, une chaîne alkylène pouvant contenir un atome d'oxygène ou de soufre ; R₁ et R₂ peuvent former ensemble un cycle à 5 ou 6 chaînons aromatiques ou non aromatiques, contenant éventuellement un ou plusieurs hétéroatomes tels qu'un atome d'azote, d'oxygène ou de soufre ;
• R₃ représente un atome d'hydrogène, un atome d'halogène, un radical alkyle en C₁-C₆, un radical cyano, un groupement aromatique, un groupement phénoxy, un radical nitro ;
• W représente un groupement CR₄ ou un atome d'azote;
• R₄ représente un atome d'hydrogène, un atome d'halogène, un radical alkyle en C₁-C₆, un radical cyano, un groupement aromatique, un radical phénoxy, un radical nitro ;
• Y représente un atome d'oxygène, un atome dé soufre, un groupement NR₅ ;
• R₅ représente un atome d'hydrogène, un radical alkyle en C₁-C₆ ;
• Z représente un groupement -CₚH₂ₚ-, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ; un groupement - C_{q}H_{2q}CO-, avec q un entier compris entre 1 et 3, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ;
• n représente un entier de 1 à 4 ;
• B représente un noyau aromatique ou hétéroaromatique, condensé ou non, comportant de 5 à16 chaînons non substitué ou substitué,
b) au moins un composé, thiolé comprenant au moins une fonction thiol (SH) et un groupement comprenant de 1 à 20 atomes de carbone, saturé ou non, ledit groupement étant éventuellement interrompu par un ou plusieurs groupes non adjacents choisis parmi -O-, -S-, -S-S-, amino (-NR-), carbonyle (-CO-), oxycarbonyle (-O-CO-), aminocarbonyle (-NR-CO-), noyau aromatique ou hétéroaromatique, les groupements amino étant ou non substitués par un ou deux radicaux alkyle en C₁-C₆ à la condition que l'atome de soufre de la fonction thiol soit rattaché audit groupement au moyen d'un atome de carbone ; ledit groupement étant éventuellement substitué par un ou plusieurs hydroxyle, alcoxy en C₁-C₆, hydroxycarbonyle, alcoxy(C₁-C₆)carbonyle, amino, aminocarbonyle, alkylcarbonylamino, dans lesquels la fonction amino est substituée ou non par un ou deux radicaux alkyle en C₁-C₆.

2. Composition selon la revendication 1, dans laquelle A est un noyau benzénique, anthracénique, naphtalénique ou quinolinique.

3. Composition selon l'une quelconque des revendications précédentes, dans laquelle A est non substitué ou substitué par un ou plusieurs groupements choisis parmi un radical alkyl(C₁-C₆)sulfonyle; un groupement pyridinium ou imidazolium non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle ; un groupement trialkyl(C₁-C₆)ammonium ; un radical sulfonate.

4. Composition selon l'une quelconque des revendications précédentes, dans laquelle X est choisi parmi un groupement CR₁R₂, avec de préférence R₁ et R₂ choisis parmi un radical alkyle en C₁-C₆.

5. Composition selon l'une quelconque des revendications précédentes, dans laquelle R₃ est choisi parmi un atome d'hydrogène.

6. Composition selon l'une quelconque des revendications précédentes, dans laquelle W est choisi parmi un groupement CR₄, avec de préférence R₄ choisi parmi un atome d'hydrogène.

7. Composition selon l'une quelconque des revendications précédentes, dans laquelle Y est choisi parmi un atome d'oxygène, ou un atome de soufre.

8. composition selon l'une quelconque des revendications précédentes, dans laquelle Z est choisi parmi un groupement -CₚH₂ₚ-, avec p un entier compris entre 2 et 4, non substitué ou substitué par un ou plusieurs substituants choisis parmi les radicaux halogéno, hydroxy, alkyle, halogénoalkyle, alcoxy, amino, mono ou dialkylamino, mono ou dihydroxyalkylamino, carboxyle.

9. Composition selon l'une quelconque des revendications précédentes, dans laquelle n est égal à 1 ou 2.

10. Composition selon l'une quelconque des revendications précédentes, dans laquelle B est un noyau benzénique, carbazolique ou indolique.

11. Composition selon l'une quelconque des revendications précédentes, dans laquelle B est non substitué ou substitué par un ou plusieurs groupements choisis parmi un radical hydroxyle ; un radical amino ; un radical di(alkyl(C₁-C₆))amino ; un radical alkyle en C₁-C₆; un radical acétamido ; un groupement pyridinium; un groupement trialkyl(C₁-C₆)ammonium.

12. Composition, selon l'une quelconque des revendications précédentes, dans laquelle le ou les composés de formule (I) sont choisis parmi les composés présentés dans le tableau ci-dessous:
| | |
|---|---|
| 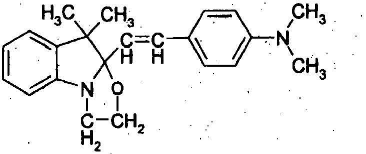 | Colorant 1 |
| 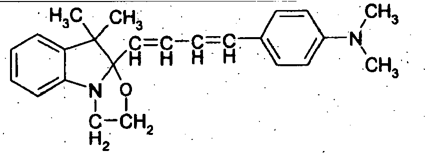 | Colorant 2 |
| 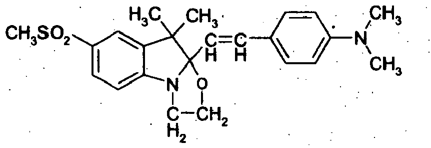 | Colorant 3 |
| 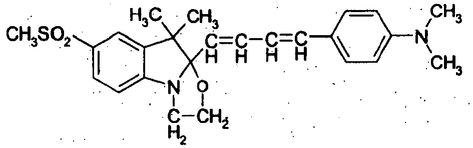 | Colorant 4 |
| 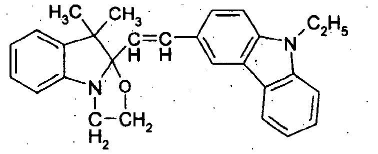 | Colorant 5 |
| 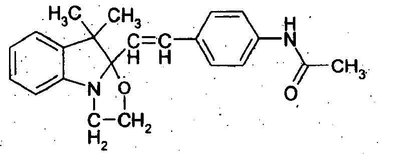 | Colorant 6 |
| 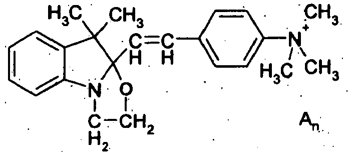 | Colorant 7 |
| 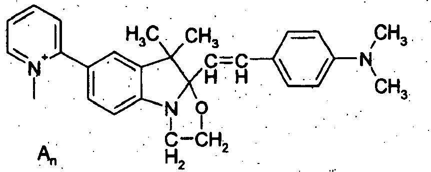 | Colorant 8 |
| 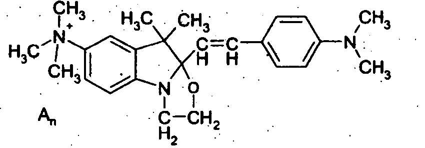 | Colorant 9 |
| 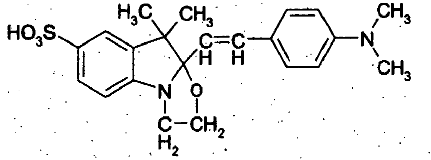 | Colorant 10 |
| 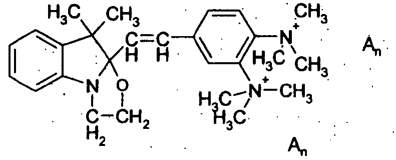 | Colorant 11 |

13. Composition selon l'une quelconque des revendications 1 à 11, dans laquelle le ou les composés de formule (I) sont choisis parmi le 3,3-diméthyl-2-(p-diméthylaminostyryl)indo)ino[1,2-b]thiazoline ; le 3,3,6-triméthyl-2-(p-diméthylaminostyryl)indolino[1,2-b]thiazoline ; le 3,3,5-triméthyl-2-(p-chlorostyryl)indolino[1,2-b]thiazoline ; le 3,3,5-triméthyl-2-[2-(thiényl)vinyl] indolino[1,2-b]thiazoline ; le 3,3-diméthyl-5-méthoxy-2-[2-(9-éthylcarbazolyl)vinyl] indolino[1,2-b]thiazoline ; le 3,3-diméthyl-5-carboéthoxy-2-(p-diméthylaminostyryl) indolino[1,2-b]thiazoline ; le 3,3-diméthyl-5-chloro-2-[2-(benzothiazolyl)vinyl)indolino [1,2-b]thiazoline ; le 3,3-diméthyl-5-méthoxy-2-(p-diméthylaminostyryl)indolino[1,2-b]thiazoline ; le 3,3-diméthyl-5-carboéthoxy-2-(3,4-méthylènedioxystyryl)indolino[1,2-b]thiazoline ; le 3,3-diméthyl-5-chloro-2-(p-méthylstyryl)indolino[1,2-b]thiazoline ; le 3,3,diméthyl-5-chloro-2-(p-méthoxystyryl)indolino[1,2-b]thiazoline; le 3,3,5-triméthyl-2-(p-acétylaminostyryl)indolino[1,2-b]thiazoline ; le 3,3-diméthyl-5-méthoxy-2-(3-hydroxy-4-méthoxystyryl)indolino[1,2-b]thiazoline ; le 3,3-diméthyl-5-carboéthoxy-2-(o-cyano styryl)indolino[1,2-b]thiazoline ; le 3,3-diméthyl-5-chloro-2-(p-diméthylaminostyryl) indolino[1,2-b]thiazoline ; le 3,3-diméthyl-5-méthylsulfonyl-2-(p-diméthylaminostyryl) indolino[1,2-b]thiazolïne ; le 3,3-diméthyl-5-phénylsulfonyl-2-(p-diméthylaminostyryl) indolino[1,2-b]thiazoline ; le 3,3-diméthyl-5-éthoxycarboxy-2-(p-diméthylaminostyryl) indolino[1,2-b]oxazoline.

14. Composition selon l'une quelconque des revendications précédentes, **caractérisée en ce que** le composé thiolé est choisi parmi l'acide thioglycotique, l'acide thiolactique, l'acide mercaptopropionique, la cystéamine, l'acide thiosuccinique, la cystéine ; l'acétylcystéine ; le glycéryl thioglycolate ; la thioglycérine ou leurs sels, seuls ou en mélanges.

15. Composition selon l'une quelconque des revendications précédentes, **caractérisée en ce que** le pH est supérieur au pKa du composé de formule (I) et inférieur ou égal à 12.

16. Procédé de traitement des fibres kératiniques, dans lequel on applique sur lesdites fibres, une composition selon l'une quelconque des revendications précédentes, pendant un temps de pose suffisant, cette application étant éventuellement suivie d'un rinçage.

17. Procédé selon la revendication 16, dans lequel on effectue un pré-traitement par action d'un agent révélateur tel que les agents acidifiants et les solvants ou la combinaison d'un ou plusieurs de ces agents révélateurs.

18. Procédé selon l'une quelconque des revendications 16 ou 17, dans lequel on effectue un post-tfaitement par action d'un agent révélateur tel que la lumière, un courant électrique, la chaleur, un agent acidifiant, un solvant, une radication électromagnétique, ou la combinaison d'un ou plusieurs de ces agents révélateurs.

19. Dispositif à plusieurs compartiments contenant dans un premier compartiment une composition tinctoriale telle que définie à l'une quelconque des revendications 1 à 13 et 15 et dans un deuxième compartiment au moins un composé thiolé tel que défini à l'une quelconque des revendications 1 ou 14.

20. Procédé d'effaçage de la coloration obtenue en mettant en oeuvre une composition selon l'une quelconque des revendications 1 à 15, **caractérise en ce que** l'on applique sur lesdites fibres une composition d'effaçage comprenant au moins un agent alcalinisant avec une teneur telle que le pH des fibres traitées soit supérieur au pKa du ou des composés de formule (I) présents dans la composition à l'origine de la coloration.

## Claims

1. Composition for dyeing keratin fibres, comprising, in a suitable dyeing medium,
a) at least one compound of formula (I), and the addition salts thereof: 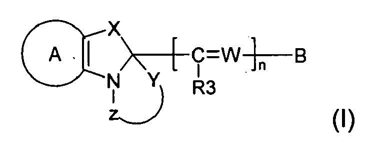 in which:
• A is a substituted or unsubstituted, 5- to 16-membered, fused or non-fused aromatic or heteroaromatic nucleus;
• X represents an oxygen atom, a sulphur atom or a group CR₁R₂;
• R₁ and R₂ represent, independently of each other, a hydrogen atom, a C₁-C₆ alkyl radical, a C₁-C₆ hydroxyalkyl radical, a C₁-C₆ alkoxyalkyl radical or an alkylene chain that may contain an oxygen or sulphur atom; R₁ and R₂ may together form an aromatic or non-aromatic 5- or 6-membered ring optionally containing one or more heteroatoms such as a nitrogen, oxygen or sulphur atom;
• R₃ represents a hydrogen atom, a halogen atom, a C₁-C₆ alkyl radical, a cyano radical, an aromatic group, a phenoxy group or a nitro radical;
• W represents a group CR₄ or a nitrogen atom;
• R₄ represents a hydrogen atom, a halogen atom, a C₁-C₆ alkyl radical, a cyano radical, an aromatic group, a phenoxy radical or a nitro radical;
• Y represents an oxygen atom, a sulphur atom or a group NRs;
• R₅ represents a hydrogen atom or a C₁-C₆ alkyl radical;
• Z represents a group -CₚH₂ₚ-, with p being an integer between 2 and 4, which may be unsubstituted or substituted with one or more substituents chosen from halo, hydroxyl, alkyl, haloalkyl, alkoxy, amino, mono- or dialkylamino, mono- or dihydroxyalkylamino and carboxyl radicals; a group -C_{q}H_{2q}CO-, with q being an integer between 1 and 3, which may be unsubstituted or substituted with one or more substituents chosen from halo, hydroxyl, alkyl, haloalkyl, alkoxy, amino, mono- or dialkylamino, mono- or dihydroxyalkylamino and carboxyl radicals;
• n represents an integer from 1 to 4;
• B represents a substituted or unsubstituted, 5- to 16-membered, fused or non-fused aromatic or heteroaromatic nucleus,
b) at least one thiol compound comprising at least one thiol function (SH) and a saturated or unsaturated group containing from 1 to 20 carbon atoms, said group being optionally interrupted with one or more nonadjacent groups chosen from -O-, -S-, -S-S-, amino (-NR-), carbonyl (-CO-), oxycarbonyl (-O-CO-), aminocarbonyl (-NR-CO-), aromatic or heteroaromatic nucleus, the amino groups being unsubstituted or substituted with one or two C₁-C₆ alkyl radicals; on condition that the sulphur atom of the thiol function is attached to said group via a carbon atom; said group being optionally substituted with one or more hydroxyl, C₁-C₆ alkoxy, hydroxycarbonyl, (C₁-C₆)alkoxycarbonyl, amino, aminocarbonyl or alkylcarbonylamino, in which the amino function is unsubstituted or substituted with one or two C₁-C₆ alkyl radicals.

2. Composition according to Claim 1, in which A is a benzene, anthracene, naphthalene or quinoline nucleus.

3. Composition according to either one of the preceding claims, in which A is unsubstituted or substituted with one or more groups chosen from a (C₁-C₆)alkylsulphonyl radical; a pyridinium or imidazolium group, which is unsubstituted or substituted with one or more substituents chosen from halo, hydroxyl, alkyl, haloalkyl, alkoxy, amino, mono- or dialkylamino, mono- or dihydroxyalkylamino and carboxyl radicals; a tri-(C₁-C₆)alkylammonium group; a sulphonate radical.

4. Composition according to any one of the preceding claims, in which X is chosen from a group CR₁R₂, R₁ and R₂ being preferably chosen from a C₁-C₆ alkyl radical.

5. Composition according to any one of the preceding claims, in which R₃ is chosen-from a hydrogen atom.

6. Composition according to any one of the preceding claims, in which W is chosen from a group CR₄, R₄ being preferably chosen from a hydrogen atom.

7. Composition according to any one of the preceding claims, in which Y is chosen from an oxygen or sulphur atom.

8. Composition according to any one of the preceding claims, in which Z is chosen from a group -CₚH₂ₚ-, with p being an integer between 2 and 4, which is unsubstituted or substituted with one or more substituents chosen from halo, hydroxyl, alkyl, haloalkyl, alkoxy, amino, monoalkylamino or dialkylamino, monohydroxyalkylamino or dihydroxyalkylamino and carboxyl radicals.

9. Composition according to any one of the preceding claims, in which n is equal to 1 or 2.

10. Composition according to any one of the preceding claims, in which B is a benzene, carbazole or indole nucleus.

11. Composition according to any one of the preceding claims, in which B is unsubstituted or substituted with one or more groups chosen from a hydroxyl radical; an amino radical; a di((C₁-C₆)alkyl)amino radical; a C₁-C₆ alkyl radical; an acetamido radical; a pyridinium group; a tri(C₁-C₆)alkylammonium group.

12. Composition according to any one of the preceding claims, in which the compound(s) of formula (I) is (are) chosen from the compounds presented in the table below:
| | |
|---|---|
| 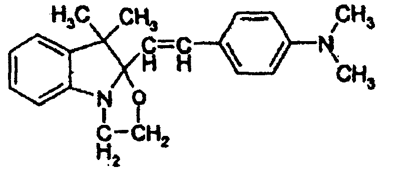 | Dye 1 |
| 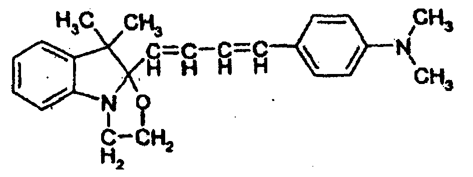 | Dye 2 |
| 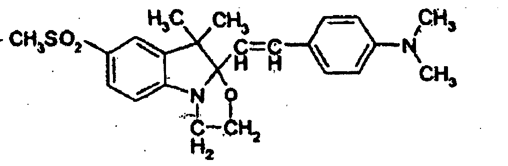 | Dye 3 |
| 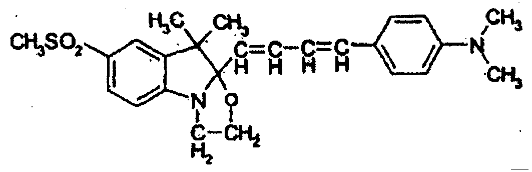 | Dye 4 |
| 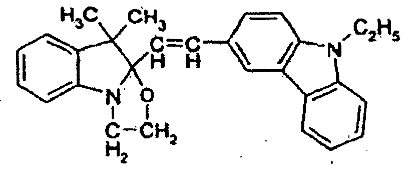 | Dye 5 |
| 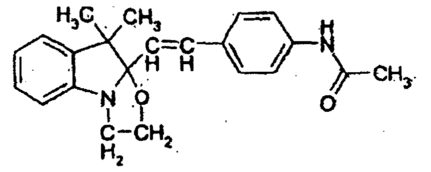 | Dye 6 |
| 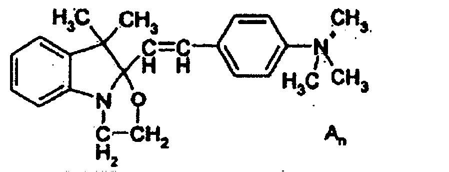 | Dye 7 |
| 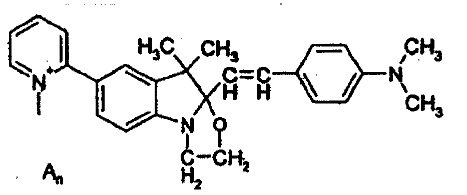 | Dye 8 |
| 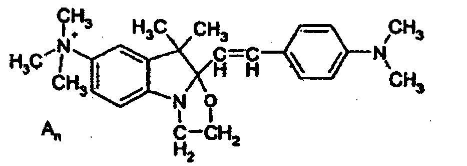 | Dye 9 |
| 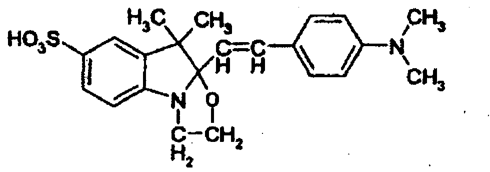 | Dye 10 |
| 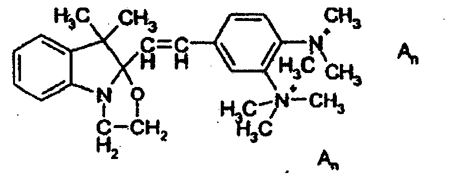 | Dye 11 |

13. Composition according to any one of Claims 1 to 11, in which the compound(s) of formula (I) is (are) chosen from 3,3-dimethyl-2-(p-dimethylaminostyryl)indolino[1,2-b]thiazoline; 3,3,5-trimethyl-2-(p-dimethylaminostyryl)indolino[1,2-b]thiazoline; 3,3,5-trimethyl-2-(p-chlorostyryl)-indolino[1,2-b]thiazoline; 3,3,5-trimethyl-2-[2-(thienyl)vinyl]indolino[1,2-b]thiazoline; 3,3-dimethyl-5-methoxy-2-[2-(9-ethylcarbazolyl)vinyl]-indolino[1,2-b]thiazoline; 3,3-dimethyl-5-carboethoxy-2-(p-dimethylaminostyryl)indolino[1,2-b]-thiazoline; 3,3-dimethyl-5-chloro-2-[2-(benzothiazolyl)vinyl]indolino[1,2-b]thiazoline; 3,3-dimethyl-5-methoxy-2-(p-dimethylaminostyryl)-indolino[1,2-b]thiazoline; 3,3-dimethyl-5-carboethoxy-2-(3,4-methylenedioxystyryl)indolino[1,2-b]thiazoline; 3,3-dimethyl-5-chloro-2-(p-methylstyryl)indolino[1,2-b]thiazoline; 3,3-dimethyl-5-chloro-2-(p-methoxystyryl]indolino[1,2-b]-thiazoline; 3,3,5-trimethyl-2-(p-acetylaminostyryl)indolino[1,2-b]thiazoline; 3,3-dimethyl-5-methoxy-2-(3-hydroxy-4-methoxystyryl)indolino[1,2-b]thiazoline; 3,3-dimethyl-5-carboethoxy-2-(o-cyanostyryl)indolino[1;2-b]thiazoline; 3,3-dimethyl-5-chloro-2-(p-dimethylaminostyryl)-indolino[1,2-b]thiazoline; 3,3-dimethyl-5-methylsulphonyl-2-(p-dimethylaminostyryl)indolino[1,2-b]thiazoline; 3,3-dimethyl-5-phenylsulphonyl-2-(p-dimethylaminostyryl)indolino[1,2-b]thiazoline; 3,3-dimethyl-5-ethoxycarboxy-2-(p-dimethylaminostyryl)indolino[1,2-b]oxazoline.

14. Composition according to any one of the preceding claims, **characterized in that** the thiol compound is chosen from thioglycolic acid, thiolactic acid, mercaptopropionic acid, cysteamine, thiosuccinic acid, cysteine; acetylcysteine; glyceryl thioglycolate; thioglycerol, or their salts, alone or as mixtures.

15. Composition according to any one of the preceding claims, **characterized in that** the pH is greater than the pKa of the compound of formula (I) and less than or equal to 12.

16. Process for treating keratin fibres, in which a composition according to any one of the preceding claims is applied to said fibres for a sufficient leave-on time, this application being optionally followed by rinsing out.

17. Process according to Claim 16, in which a pretreatment is performed through the action of a revealing agent such as acidifying agents and solvents or the combination of one or more of these revealing agents.

18. Process according to either one of Claims 16 and 17, in which a post-treatment is performed through, the action of a revealing agent such as light, an electrical current, heat, an acidifying agent, a solvent, an electromagnetic radiation or the combination of one or more of these revealing agents.

19. Multicompartment device containing in a first compartment a dye composition as defined in any one of Claims 1 to 13 and 15 and in a second compartment at least one thiol compound as defined in either one of Claims 1 and 14.

20. Process for effacing the coloration obtained using a composition according to any one of Claims 1 to 15, **characterized in that** an effacing composition comprising at least one basifying agent is applied to said fibres, in a content such that the pH of the treated fibres is greater than the pKa of the compound(s) of formula (I) present in the composition giving rise to the coloration.

## Patentansprüche

1. Zusammensetzung zum Färben von Keratinfasern, die in einem zum Färben geeigneten Medium enthält:
a) mindestens eine Verbindung der Formel (I) und ihre Additionssalze: 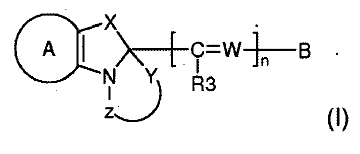 in der Formel:
A bedeutet einen 5- bis 16-gliedrigen, aromatischen oder heterocyclischen, kondensierten oder nicht kondensierten, substituierten oder unsubstituierten Kern;
X bedeutet ein Sauerstoffatom, ein Schwefelatom oder eine Gruppe CR₁R₂;
R₁ und R₂ bedeuten unabhängig voneinander ein Wasserstoffatom, eine Alkyl(C₁₋₆)gruppe, eine Hydroxyalkyl(C₁₋₆)gruppe, eine Alkoxyalkyl(C₁₋₆)gruppe, eine Alkylenkette, die ein Sauerstoffatom oder ein Schwefelatom enthalten kann; R₁ und R₂ können gemeinsam einen 5- oder 6-gliedrigen, aromatischen oder nichtaromatischen Ring bilden, der gegebenenfalls ein oder mehrere Heteroatome enthält, wie ein Stickstoffatom, ein Sauerstoffatom oder ein Schwefelatom;
R₃ bedeutet ein Wasserstoffatom, ein Halogenatom, eine Alkyl(C₁₋₆)gruppe, eine Cyanogruppe, eine aromatische Gruppe, eine Phenoxygruppe, eine Nitrogruppe;
W bedeutet eine Gruppe CR₄ oder ein Stickstoffatom;
R₄ bedeutet ein Wasserstoffatom, ein Halogenatom, eine Alkyl(C₁₋₆)gruppe, eine Cyanogruppe, eine aromatische Gruppe, eine Phenoxygruppe, eine Nitrogruppe;
Y bedeutet ein Sauerstoffatom, ein Schwefelatom, eine Gruppe NR₅;
R₅ bedeutet ein Wasserstoffatom, eine Alkyl(C₁₋₆)gruppe;
Z bedeutet eine Gruppe -CₚH₂ₚ-, wobei p eine ganze Zahl im Bereich von 2 bis 4 bedeutet, die unsubstituiert ist oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carbon ausgewählt sind; eine Gruppe - C_{q}H_{2q}CO-, wobei q eine ganze Zahl im Bereich von 1 bis 3 bedeutet, die unsubstituiert ist oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind;
n bedeutet eine ganze Zahl von 1 bis 4;
B bedeutet einen 5- bis 16-gliedrigen, aromatischen oder heterocyclischen, kondensierten oder nicht kondensierten, substituierten oder unsubstituierten Kern;
b) mindestens eine Thiolverbindung, die mindestens eine Thiofunktion (SH) aufweist und eine gesättigte oder ungesättigte Gruppe mit 1 bis 20 Kohlenstoffatomen enthält, die gegebenenfalls durch eine oder mehrere, nicht aneinander angrenzende Gruppen unterbrochen ist, die unter -O-, -S-, -S-S-, Amino (-NR-), Carbonyl (-CO-), Oxycarbonyl (-O-CO-), Aminocarbonyl (-NR-CO-), einem aromatischen oder heteroaromatischen Kern ausgewählt sind, wobei die Aminogruppen gegebenenfalls mit einer oder mit zwei C₁₋₆-Alkylgruppen substituiert sind; mit der Maßgabe, dass das Schwefelatom der Thiofunktion über ein Kohlenstoffatom an die Gruppe gebunden ist; wobei die Gruppe gegebenenfalls mit einer oder mehreren Hydroxygruppen, Alkoxy(C₁₋₆)gruppen, Hydroxycarbonylgruppen, Alkoxy(C₁₋₆)carbonylgruppen, Aminogruppen, Aminocarbonylgruppen, Alkylcarbonylgruppen substituiert ist, bei denen die Aminofunktion gegebenenfalls mit einer oder mit zwei C₁₋₆-Alkylgruppen substituiert ist.

2. Zusammensetzung nach Anspruch 1, wobei A ein Benzolring, ein Anthracenring, ein Naphthalinring oder ein Chinolinring ist.

3. Zusammensetzung nach einem der vorhergehenden Ansprüche, wobei A unsubstituiert vorliegt oder mit einer oder mehreren Gruppen substituiert ist, die unter einer Alkylsulfonyl(C₁₋₆)-gruppe; einer Pyridinium- oder Imidazoliumgruppe, die unsubstituiert ist oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind; einer Trialkyl(C₁₋₆)ammoniumgruppe; einer Sulfonatgruppe ausgewählt sind.

4. Zusammensetzung nach einem der vorhergehenden Ansprüche, wobei X unter den Gruppen CR₁R₂ ausgewählt ist, wobei die Gruppen R₁, und R₂ vorzugsweise unter den Alkyl(C₁₋₆)gruppen ausgewählt sind.

5. Zusammensetzung nach einem der vorhergehenden Ansprüche, wobei die Gruppe R₃ unter einem Wasserstoffatom ausgewählt ist.

6. Zusammensetzung nach einem der vorhergehenden Ansprüche, wobei W unter den Gruppen CR₄ ausgewählt ist, wobei die Gruppe R₄ vorzugsweise unter einem Wasserstoffatom ausgewählt ist.

7. Zusammensetzung nach einem der vorhergehenden Ansprüche, wobei Y unter einem Sauerstoffatom oder einem Schwefelatom ausgewählt ist.

8. Zusammensetzung nach einem der vorhergehenden Ansprüche, wobei Z unter einer Gruppe -CₚH₂ₚ- ausgewählt ist, wobei p eine ganze Zahl von 2 bis 4 bedeutet, die unsubstituiert ist oder mit einem oder mehreren Substituenten substituiert ist, die unter den Substituenten Halogen, Hydroxy, Alkyl, Halogenalkyl, Alkoxy, Amino, Mono- oder Dialkylamino, Mono- oder Dihydroxyalkylamino, Carboxy ausgewählt sind.

9. Zusammensetzung nach einem der vorhergehenden Ansprüche, wobei n 1 oder 2 bedeutet.

10. Zusammensetzung nach einem der vorhergehenden Ansprüche, wobei B einen Benzolring, einen Carbazolring oder einen Indolring bedeutet.

11. Zusammensetzung nach einem der vorhergehenden Ansprüche, wobei B unsubstituiert vorliegt oder mit einem oder mehreren Substituenten substituiert ist, die unter einer Hydroxygruppe; einer Aminogruppe; einer Di(alkyl(C₁₋₆))aminogruppe; einer Alkyl(C₁₋₆)gruppe; einer Acetamidogruppe; einer Pyridiniumgruppe; einer Trialkyl(C₁₋₆)ammoniumgruppe ausgewählt sind.

12. Zusammensetzung nach einem der vorhergehenden Ansprüche, wobei die Verbindung(en) der Formel (I) unter den in der folgenden Tabelle dargestellten Verbindungen ausgewählt sind:
| | |
|---|---|
| 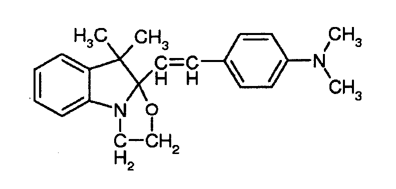 | Farbstoff 1 |
| 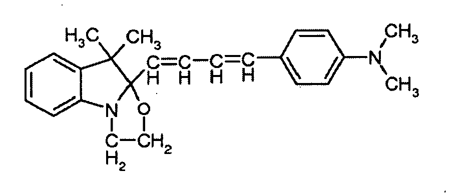 | Farbstoff 2 |
| 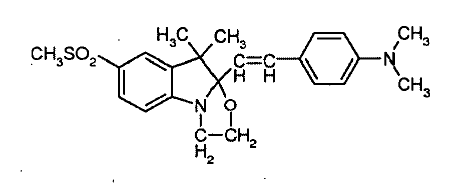 | Farbstoff 3 |
| 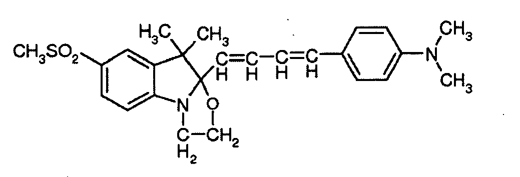 | Farbstoff 4 |
| 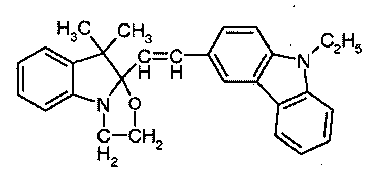 | Farbstoff 5 |
| 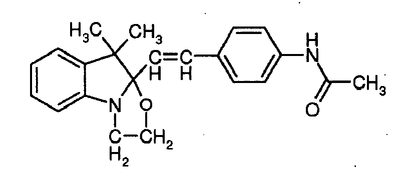 | Farbstoff 6 |
| 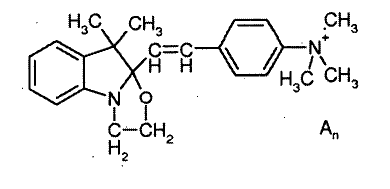 | Farbstoff 7 |
| 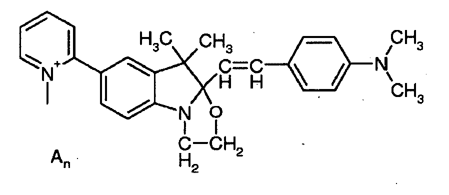 | Farbstoff 8 |
| 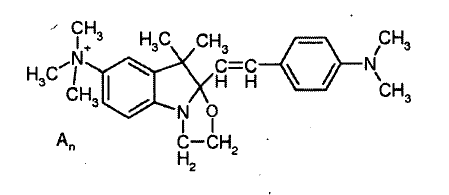 | Farbstoff 9 |
| 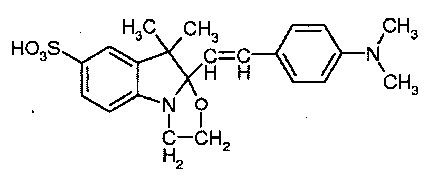 | Farbstoff 10 |
| 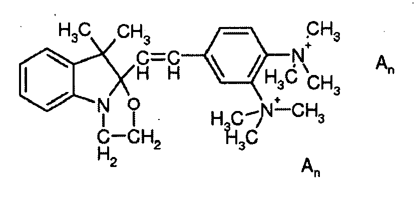 | Farbstoff 11 |

13. Zusammensetzung nach einem der Ansprüche 1 bis 11, wobei die Verbindung(en) der Formel (I) unter 3,3-Dimethyl-2-(p-dimethylaminostyryl)indolino[1,2-b]thiazolin; 3,3,5-Trimethyl-2-(p-dimethylaminostyryl)indolino[1,2-b]thiazolin; 3,3,5-Trimethyl-2-(p-chlorstyryl)indolino[1,2-b]thiazolin; 3,3,5-Trimethyl-2-[2-(thienyl)vinyl]indolino[1,2-b]thiazolin; 3,3-Dimethyl-5-methoxy-2-[2-(9-ethylcarbazolyl)vinyl]indolino[1,2-b]thiazolin; 3,3-Dimethyl-5-carboethoxy-2-(p-dimethylaminostyryl)indolino[1,2-b]thiazolin; 3,3-Dimethyl-5-chlor-2-[2-(benzothiazolyl)vinyl)indolino[1,2-b]thiazolin; 3,3-Dimethyl-5-methoxy-2-(p-dimethylaminostyryl)-indolino[1,2-b]thiazolin; 3,3-Dimethyl-5-carboethoxy-2-(3,4-methylendioxystyryl)indolino[1,2-b]thiazolin; 3,3-Dimethyl-5-chlor-2- (p-methylstyryl)indolino[1,2-b]thiazolin; 3,3-Dimethyl-5-chlor-2-(p-methoxystyryl)indolino[1,2-b]thiazolin; 3,3,5-Trimethyl-2-(p-acetylaminostyryl)indolino[1,2-b]thiazolin; 3,3-Dimethyl-5-methoxy-2-(3-hydroxy-4-methoxystyryl)indolino[1,2-b]-thiazolin; 3,3-Dimethyl-5-carboethoxy-2-(o-cyano-styryl)-indolino[1,2-b]thiazolin; 3,3-Dimethyl-5-chlor-2-(p-dimethylaminostyryl)indolino[1,2-b]thiazolin; 3,3-Dimethyl-5-methylsulfonyl-2-(p-dimethylaminostyryl)indolino[1,2-b]thiazolin; 3,3-Dimethyl-5-phenylsulfonyl-2-(p-dimethylaminostyryl)-indolino[1,2-b]thiazolin; 3,3-Dimethyl-5-ethoxycarboxy-2-(p-dimethylaminostyryl)indolino[1,2-b]oxazolin ausgewählt sind.

14. Zusammensetzung nach einem der vorhergehenden Ansprüche, **dadurch gekennzeichnet, dass** die Thiolverbindung einzeln oder in Form eines Gemisches unter Thioglycolsäure, Thiomilchsäure, Mercaptopropionsäure, Cysteamin, Thiobernsteinsäure, Cystein; Acetylcystein; Glycerylthioglycolat; Thioglycerin oder ihren Salzen ausgewählt ist.

15. Zusammensetzung nach einem der vorhergehenden Ansprüche, **dadurch gekennzeichnet, dass** der pH-Wert über dem pKs-Wert der Verbindung der Formel (I) liegt und kleiner oder gleich 12 ist.

16. Verfahren zur Behandlung von Keratinfasern, bei dem eine Zusammensetzung nach einem der vorhergehenden Ansprüche während einer ausreichenden Einwirkzeit auf die Keratinfasern aufgebracht wird, wobei nach der Anwendung gegebenenfalls gespült wird.

17. Verfahren nach Anspruch 16, bei dem durch Einwirkung eines Entwicklers, wie Alkalisierungsmitteln und Lösemitteln, oder der Kombination aus einem oder mehreren dieser Entwickler eine Vorbehandlung durchgeführt wird.

18. Verfahren nach einem der Ansprüche 16 oder 17, bei dem durch Einwirkung eines Entwicklers, wie Licht, elektrischen Strom, Wärme, eines Ansäuerungsmittels, eines Lösungsmittels, elektromagnetischer Strahlung oder der Kombination aus einem oder mehreren dieser Entwickler eine Nachbehandlung durchgeführt wird.

19. Vorrichtung mit mehreren Abteilungen, die in einer ersten Abteilung eine Farbmittelzusammensetzung, wie sie in einem der Ansprüche 1 bis 13 und 15 definiert ist, und in einer zweiten Abteilung mindestens eine Thiolverbindung enthält, wie sie in einem der Ansprüche 1 oder 14 definiert ist.

20. Verfahren zum Abnehmen der durch Verwendung einer Zusammensetzung nach einem der Ansprüche 1 bis 15 gebildeten Farbe, **dadurch gekennzeichnet, dass** eine Zusammensetzung, die mindestens ein Alkalisierungsmittel in einer solchen Menge enthält, dass der pH-Wert der behandelten Fasern über dem pKs-Wert der in der ursprünglichen Farbmittelzusammensetzung enthaltenen Verbindung(en) der Formel (I) liegt, auf die Fasern aufgebracht wird.
